(19) **Europäisches Patentamt / European Patent Office / Office européen des brevets**

(11) **EP 2 096 567 A1**

(12) **EUROPEAN PATENT APPLICATION**
published in accordance with Art. 153(4) EPC

(43) Date of publication:
**02.09.2009 Bulletin 2009/36**

(51) Int Cl.:
**G06F 19/00** [(2006.01)]     **G06Q 10/00** [(2006.01)]
**C12N 15/09** [(2006.01)]

(21) Application number: **07850579.9**

(22) Date of filing: **13.12.2007**

(86) International application number:
**PCT/JP2007/074063**

(87) International publication number:
**WO 2008/072713 (19.06.2008 Gazette 2008/25)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **14.12.2006 JP 2006337629**

(71) Applicant: **Takeda Pharmaceutical Company Limited**
**Osaka-shi,**
**Osaka 541-0045 (JP)**

(72) Inventor: **NAKAMURA, Shingo**
**Tsukuba-shi**
**Ibaraki 300-4293 (JP)**

(74) Representative: **Jones, Nicholas Andrew et al**
**Withers & Rogers LLP**
**Goldings House**
**2 Hays Lane**
**GB-London SE1 2HW (GB)**

(54) **METHOD OF ESTIMATING SECONDARY STRUCTURE IN RNA AND PROGRAM AND APPARATUS THEREFOR**

(57) Assuming frame F having specific short length L2 on a transcript, a structure in a small-section is sequentially analyzed while shifting the frame F finely in a step-like manner by constant value t, and first probability for a specific secondary structure in a specific position in each small-section is determined. Then, at least one specific position proved in each small-section is arranged in corresponding position on the original transcript, and the magnitude of degree of overlapping between the specific positions is determined as second probability. By focusing on these two probabilities, it is possible to predict whether an intended secondary structure is actually present in RNA such as mRNA with higher reliability.

FIG. 1

## Description

## Technical field

The present invention relates to a technique of predicting presence of local secondary structure in RNA, and a method of the same, a program for making a computer execute the method, and an apparatus for executing the method.

## (Background of the invention)

mRNA largely changes its form (secondary and tertiary structures) as a whole depending on an event in a cell. A general structure of mRNA can not be determined uniquely during synthesis of mRNA in a nucleus, when mRNA is being spliced, when mRNA is transported outside a nucleus, during translation into a protein, after the translation, and so on.
On the other hand, since RNA is a macromolecule that is liable to interact autologously, it can be presumed that RNA forms some higher-order structure. However, in long RNA, there is little difference in energy between the most stable structure and a group of sub-most stable structures, so that such long RNA must be present in a solution as a mixture of a plurality of secondary and tertiary structures (in other words, a unique structure is not ensured). Due to these two factors, it was impossible to grasp the entire higher-order structures of mRNA, and influence of such structures on biological phenomenon has been little aware of.

In the field of structural biology, structures of tRNA and rRNA which are functional RNAs have been actively researched heretofore. However, for mRNA which is greatly recognized as functioning as a "messenger" for translation of genetic information of DNA which is gene into a protein, higher-order structures thereof have been little noticed heretofore. This is because mRNA does not have any remarkable function.
From recent researches, however, it has been gradually revealed that a local secondary structure on mRNA is involved in molecular biological events such as edition of RNA, riboswitch, IRES and frame shift. Also, involvement of such a local secondary structure in difference in efficiency of RNAi and antisense pharmaceuticals depending on the binding site, and binding site of RNAi can be suggested. That is, it has been revealed that even for higher-order structure of mRNA of which entire structure cannot be grasped, a local higher-order structure thereof has a biological function when an object is limited to mRNA corresponding to a certain intracellular event. However, the mRNA reported "to have a structure and to have a function" as described above is very few, and secondary structures of mRNA which can be a target of drug discovery are very limited.

The present inventors previously developed software capable of comprehensively searching local secondary structures in mRNA molecules from cDNA database using a computer based on the idea that every mRNA can form some higher-order structure stochastically based on the tendency of self-interaction which is a characteristic of RNA molecules, and such a structure has not been recognized as a structure so far because it lacks a special function in a living body or is not stable enough to exert a function (Patent document 1). Further, we demonstrated that a local secondary structure that is extracted by such searching could exist relatively stably in a living body (in other words, more easily stabilized by a specific bond with a certain substance (for example, a low molecular weight compound)) using an in vitro experimental system. This result strongly suggests the possibility of drug discovery targeting secondary structures of mRNA which would be an alternative for conventional antisense pharmaceuticals or RNAi pharmaceuticals (complementary to primary structure of mRNA) (that is, screening of a compound that suppresses translation of mRNA into a protein by binding to the secondary structure part or the vicinity thereof to stabilize the structure (or enhances translation efficiency by improving degradation stability of mRNA)).

As described above, while it has been theoretically revealed that every mRNA is able to form some secondary structure locally in a molecule, for realizing more effective development and design of pharmaceuticals targeting the secondary structure of mRNA (for example, antisense nucleic acid, RNAi, low molecular weight compound and the like), a means that accurately predicts whether the objective mRNA actually forms the extracted local secondary structure in a living body is demanded. It is to be noted that such long-chain RNA is not ensured to uniquely form the most stable structure.

For the above demand, the conventional RNA structure prediction software including "mfold" (GCG Software; see Non-patent document 1) has such a drawback that premise for prediction is diverged from intracellular condition, and a reliable result is not obtained only by simple application to long-chain RNA such as mRNA, and it is often the case that a plurality of local secondary structure candidates are merely recited in parallel manner to lead confusion, and hence, it is impossible to discuss a local secondary structure that can be present in a living body simply based on such a prediction result. Originally, existing secondary structure prediction software such as mfold is designed based on the requirement that how accurate a structure of relatively short RNA having a unique secondary structure is predicted, and fails to coincide as it is inherently with the present requirement that the structure of RNA capable of forming a plurality of structures is predicted.

Since the software disclosed in Patent document 1 is intended to search and extract intended secondary structures comprehensively by pattern matching from data set of the primary structure (base sequence) of mRNA, it does not necessarily provide sufficient suggestion about the likelihood of actual presence of the extracted secondary structure in a living body.

patent document 1: WO2006/054788
patent document 2: WO2004/011610
non-patent document 1: Proc. Natl. Acad. Sci. USA, 86: 7706-10 (1989)

**Disclosure of the Invention**

**Problems to be Solved by the Invention**

It is an object of the present invention to provide a novel method capable of predicting whether an intended secondary structure actually exists in RNA such as mRNA with higher reliability, and a program for making a computer execute the method, and an apparatus for executing the method.

**Means for solving the problems**

As a result of diligent effort for resolving the above object, the present inventors devised a new method which includes: assuming a frame of a certain short length in a certain position on an objective section having a long sequence such as mRNA, performing structure extraction in a small-section defined by the frame, performing structure extraction in each small-section while shifting the frame by a very small length (sufficiently shorter than the frame length), and overlapping the extraction results on the original objective section. The present inventors found that, thorough the method of returning and overlapping results analyzed in individual small-sections defined so as to overlap with each other onto the original objective section, positions of an objective secondary structure that are extracted in individual small-sections have correlation with each other and the degree of the correlation can be a new index indicating likelihood of presence of the secondary structure, and accomplished the present invention.

Specifically, the present invention has the following features.

(1) A method of predicting whether a specific secondary structure having desired characteristic parameter values is present in RNA, comprising the steps of:

(A) selecting an objective section having entire length of L1 to be predicted from the entire length of RNA, and assuming a frame having entire length of L2 (L2<L1) which is longer than a sequence length of the specific secondary structure,
in a small-section S1 which is defined to cover one frame from one end of the objective section, extracting a small structure which includes the specific secondary structure and can be formed by RNA, based on Gibbs free energy as an index for extraction and examining at which position in the extracted small structure, the specific secondary structure is present, thereby determining at what probability the specific secondary structure is present in each of at least one specific position X1 in the small-section S1 and making it as first probability $Dg(X1)$ for each specific position X1 in the small-section S1,
repeating the operation of defining a next small-section by shifting the frame toward the other end by constant length t ($t<L2$) and determining first probability for each specific position in the small-section similarly to that of the small-section S1 until the frame reaches the other end of the objective section, thereby obtaining first probabilities $Dg(X1)$ to $Dg(Xn)$ for each specific position in each small-section from the first small-section S1 to the last small-section Sn;
(B) applying each specific position determined for each small-section in the step of (A) to a corresponding position on the objective section, thereby determining at which position and at what degree on the objective section, each specific position overlaps, and making the degree of overlapping for each position on the objective section as second probability; and
(C) predicting presence of the specific secondary structure in the objective section based on the first probability and the second probability determined in the steps (A) and (B).

(2) The method according to the above (1), wherein the entire length L1 of the objective section is 100 or more bases.
(3) The method according to the above (1) or (2), wherein the specific secondary structure is a stem-loop structure, and the characteristic parameter values defining the structure include stem length, loop length, and an allowable number of mismatches in the stem-loop.

(4) The method according to any one of the above (1) to (3), wherein the specific secondary structure is a recess-shaped structure having an interspace existing between two stem-loop structures and not interacting with other base sequences, and respective inner stem parts of the stem-loop structures on both sides of the interspace, and the characteristic parameter values defining the structure include a number of bases in the interplace, and a sum of numbers of bases of the respective stem inner parts of the stem-loop structures on both sides.

(5) The method according to any one of the above (1) to (4), wherein a number of bases of the entire length L2 of the frame is 50 to 300.

(6) The method according to any one of the above (1) to (5), wherein a number of bases of the constant length t is 1 to 10.

(7) The method according to any one of the above (1) to (6), wherein in each small-section, extracting a small structure having Gibbs free energy of a predetermined value or less.

(8) The method according to any one of the above (1) to (7), wherein in the step (C), the first probability and the second probability are concurrently displayed in a graph in which the objective section corresponds to one axis, and the presence of the specific secondary structure in the objective section is predicted based on the first probability and the second probability displayed in the graph.

(9) The method according to the above (8), wherein the graph is a bar graph represented on an orthogonal coordinate plane in which the objective section corresponds to one axis and the value of the first probability corresponds to the other axis, and magnitude of the value of the first probability is displayed in correspondence with the length of the bar in the graph, and magnitude of the value of the second probability is displayed by changing color and/or pattern of the part where bars of the graph overlap with each other.

(10) The method according to the above (8) or (9), wherein for a sequence of the extracted specific secondary structure, calculating a value of Gibbs free energy of the specific secondary structure itself, and displaying the value in a superimposed manner on the graph.

(11) The method according to any one of the above (8) to (10), wherein for the extracted specific secondary structure, a base usually not forming a base pair, and
a base not forming a base pair in one specific secondary structure but participating in formation of a base pair in other specific secondary structure are displayed in a superimposed manner in the graph so that they are discriminable from each other.

(12) A computer program for predicting whether a specific secondary structure having intended characteristic parameter values is present in RNA, the program making a computer function as

(P1) an input means that inputs at least primary structure data of an objective section which is to be an object for prediction at least in primary structure of RNA, characteristic parameter values of the specific secondary structure, length L2 of a frame assumed as a section which is shorter than entire length L1 of the objective section and longer than a sequence length of the specific secondary structure, and constant length t(t<L2) which is a shifting amount of the frame;

(P2) a first calculation means that extracts a plurality of small structures which include the specific secondary structure and can be formed by RNA, based on Gibbs free energy as an index for extraction in a small-section S1 which is set to cover one frame from one end of the objective section, and examines at which position in the extracted small structure, the specific secondary structure is present, thereby determining at what probability the specific secondary structure is present in each of at least one specific position X1 in the small-section S1 and making it as first probability Dg(X1) for each specific position X1 in the small-section S1, and repeats the operation of defining a next small-section by shifting the frame toward the other end by the constant length t (t<L2) and determining first probability for each specific position in the small-section similarly to that of the small-section S1, until the frame reaches the other end of the objective section, thereby obtaining first probabilities Dg(X1) to Dg(Xn) for each specific position in each small-section from the first small-section S1 to the last small-section Sn;

(P3) a second calculation means that applies each specific position determined for each small-section by the first calculation means to a corresponding position on the objective section, thereby determining at which position and at what degree on the objective section, each specific position overlaps, and making the degree of overlapping for each position on the objective section as second probability; and

(P4) an output means that outputs the first probability determined by the first calculation means and the second probability determined by the second calculation means.

(13) The program according to the above (12), wherein the entire length L1 of the objective section is 100 or more bases.

(14) The program according to the above (12) or (13), wherein the specific secondary structure is a stem-loop structure, and the characteristic parameter values defining the structure include stem length, loop length, and an allowable number of mismatches in the stem-loop.

(15) The program according to any one of the above (12) to (14), wherein the specific secondary structure is a recess-shaped structure having an interplace existing between two stem-loop structures and not interacting with other base sequences, and respective inner stem parts of the stem-loop structures on both sides of the interplace, and the characteristic parameter values defining the structure include a number of bases in the interplace, and a sum of numbers of bases of the respective stem inner parts of the stem-loop structures on both sides.

(16) The program according to any one of the above (12) to (15), wherein in each small-section, extracting a small structure having Gibbs free energy of a predetermined value or less.

(17) The program according to any one of the above (12) to (16), wherein the output means outputs the first probability and the second probability so that they are concurrently displayed in a graph in which the objective section corresponds to one axis.

(18) The program according to the above (17), wherein the graph is a bar graph represented on an orthogonal coordinate plane in which the objective section corresponds to one axis and the value of the first probability corresponds to the other axis, and magnitude of the value of the first probability is displayed in correspondence with the length of the bar in the graph, and magnitude of the value of the second probability is displayed by changing color and/or pattern of the part where bars of the graph overlap with each other.

(19) The program according to the above (17) or (18), wherein for a sequence of the extracted specific secondary structure, a value of Gibbs free energy of the specific secondary structure itself is calculated, and the value is displayed in a superimposed manner on the graph.

(20) The program according to any one of the above (17) to (19), wherein for the extracted specific secondary structure,

a base usually failing to form a base pair, and

a base failing to form a base pair in one specific secondary structure but participating in formation of a base pair in other specific secondary structure are displayed in a superimposed manner in the graph so that they are discriminable from each other.

(21) An apparatus for predicting whether a specific secondary structure having intended characteristic parameter values is present in RNA, comprising

(M1) an input unit that inputs at least primary structure data of objective section which is to be an object for prediction at least in primary structure of RNA, characteristic parameter values of the specific secondary structure, length L2 of a frame assumed as a section which is shorter than entire length L1 of the objective section and longer than a sequence length of the specific secondary structure, and constant length t(t<L2) which is a shifting amount of the frame;

(M2) a first calculation unit that extracts a plurality of small structures which include the specific secondary structure and can be formed by RNA, based on Gibbs free energy as an index for extraction in a small-section S1 which is set to cover one frame from one end of the objective section, and examines at which position in the extracted small structure, the specific secondary structure is present, thereby determining at what probability the specific secondary structure is present in each of at least one specific position X1 in the small-section S1 and making it as first probability Dg(X1) for each specific position X1 in the small-section S1, and

repeats the operation of defining a next small-section by shifting the frame toward the other end by the constant length t (t<L2) and determining first probability for each specific position in the small-section similarly to that of the small-section S1 until the frame reaches the other end of the objective section, thereby obtaining first probabilities Dg(X1) to Dg(Xn) for each specific position in each small-section from the first small-section S1 to the last small-section Sn;

(M3) a second calculation unit that applies each specific position determined for each small-section by the first calculation unit to a corresponding position on the objective section, thereby determining at which position and at what degree on the objective section, each specific position overlaps, and making the degree of overlapping for each position on the objective section as second probability; and

(M4) an output unit that outputs the first probability determined by the first calculation unit and the second probability determined by the second calculation unit.

(22) The apparatus according to the above (21), wherein the entire length L1 of the objective section is 100 or more bases.

(23) The apparatus according to the above (21) or (22), wherein the specific secondary structure is a stem-loop structure, and the characteristic parameter values defining the structure include stem length, loop length, and an allowable number of mismatches in the stem-loop.

(24) The apparatus according to any one of the above (21) to (23), wherein the specific secondary structure is a recess-shaped structure having an interplace existing between two stem-loop structures and not interacting with other base sequences, and respective inner stem parts of the stem-loop structures on both sides of the interplace, and the characteristic parameter values defining the structure include a number of bases in the interplace, and a sum of numbers of bases of the respective stem inner parts of the stem-loop structures on both sides.

(25) The apparatus according to any one of the above (21) to (23), wherein at least the first calculation unit and the second calculation unit are a computer.

(26) The apparatus according to the above (25), wherein the input unit is included in the computer, and the primary structure data of the objective section is inputted by transferring the primary structure data stored in a storage device in or outside the computer in response to an instruction made by an operator who operates the computer, and the characteristic parameter values of a specific secondary structure, the frame length L2, and the constant length t are inputted by the operator who operates the computer.

**Effects of the invention**

As described above, in the conventional prediction method, even if an objective section is set to be short for a long transcript of RNA, extraction of an actual structure from a large number of secondary structures that can be formed is only by an operation of making a choice by mainly referring values of Gibbs free energy. Such extraction is based on the idea of focusing on only the most stable structure based on the premise that small Gibbs free energy means high possibility of presence.

In contrast, in the present invention, first, a small-section is defined on a transcript while assuming a specific short frame, and a small structure containing a specific secondary structure is extracted in each small-section while shifting the frame finely in a step-like manner (that is, shifting so that the frame after shifting is sufficiently overlapped with the frame before shifting). The extraction itself of the small structure in such a small-section is extraction according to stability based on a value of Gibbs free energy as is the case with the conventional art.

In the present invention, at which position in the extracted small structure, the specific secondary structure is present is examined, whereby "at what probability the specific secondary structure is present in each specific position in each small-section" is determined, and it is made as first probability.

The first probability is given individually to every specific position proved in the extraction of the specific secondary structure in each small-section, and is "an index that indicates at what probability the specific secondary structure is present in each specific position".

When the first probability is high, it means that an intended specific secondary structure is present in the specific position more certainly. However, there is also a case where no small structure is extracted in a particular small-section. In such a case, there is no specific position in that small-section, and the first probability is made as 0. There is also the case where only one small structure is outputted for the small-section in a particular prediction method or in a particular sequence of small-section. In such a case, if at least one specific secondary structures observed in the small-section satisfy the parameter, the first probability for these specific secondary structures can be made as being equally high.

In any cases, since such first probability is probability of structure only in a small-section, even for a specific position exhibiting highest first probability in one small-section, there is a case where the specific secondary structure is not actually present when structure in long RNA is examined in an actual experiment.

The major reason is that, for a certain specific sequence to form a certain secondary structure, it is important that a sequence that can more strongly interact with the specific sequence is not present near the specific sequence (environment where the specific sequence is present), in addition to a base sequence of the specific sequence.

Therefore, when such a sequence that can interact is forcibly excluded by separation into small-sections, even if the sequence exhibits good first probability in prediction, it does not reflect the actual case. In particular, the one existing near both ends of the small-section can be outputted as a so-called pseudo structure.

Since a number of candidates exhibiting high first probability is large, it is necessary to conduct further focusing on discussing probability of the specific secondary structure.

Next, in the present invention, each specific position determined for each of a plurality of small-sections is applied to a corresponding position of the original objective section. The operation of covering the whole of a long objective section while overlapping and correlating each extraction result for each small-section is an important feature of the present invention employing the concept of frame shifting.

When such an operation is performed, between small-sections that overlap with each other, the respective specific positions overlap with each other, and the overlapping is not uniform, and the degree of overlapping differs depending on the position. This phenomenon is important, and the present invention newly revealed that the specific position showing larger overlaps is more likely to be present actually. The degree of overlapping between the specific positions is made as second probability in the present invention.

The second probability is "an index that indicates each specific position itself has what extent of credibility" obtained by correlating the specification positions proved in each small-section, and is a value given to each specific position similar to the first probability.

When the second probability is high, the credibility that the specific position itself is a position of the specific secondary structure is high.

Therefore, a specific position having high first probability and high second probability has high credibility that the specific position itself is a position of the specific secondary structure, and the specific secondary structure is present there more certainly, so that it becomes possible to quantitatively predict whether the objective specific secondary structure is present at significant reliability.

**Brief explanation of the drawings**

[Fig. 1] is a schematic view showing each step and principle of prediction of the method of the present invention in an easily recognizable manner.

[Fig. 2] is a view showing examples of small structures extracted by structure prediction in a small-section.

[Fig. 3] is a block diagram schematically showing one exemplary configuration of the apparatus of the present invention.

[Fig. 4] is a view showing a flow of the program of the present invention.

[Fig. 5] is a view showing a preferred example of display design when a prediction result by the present invention is outputted, and is a graph chart showing a prediction result according to Example of the present invention.

[Fig. 6] is a partially enlarged view of a small bar graph representing presence or absence of a free base or a pseudo-free base taken out only from the graph of Fig. 5.

[Fig. 7] is a graph chart showing a structure prediction result for a stem-loop structure in Example 2 of the present invention.

[Fig. 8] is a graph showing a structure prediction result for a recess-shaped structure in Example 2 of the present invention.

[Fig. 9] is a graph chart showing a visualized structure prediction result R1144(75) for a stem-loop structure in Example 2 of the present invention.

[Fig. 10] is a graph chart showing a visualized structure prediction result R1814(75) for a stem-loop structure in Example 2 of the present invention.

[Fig. 11] is a graph chart showing a visualized structure prediction result R1865(75) for a stem-loop structure in Example 2 of the present invention.

[Fig. 12] is a graph showing a structure prediction result R-1144(75) for a recess-shaped structure in Example 2 of the present invention.

[Fig. 13] is a graph chart showing a visualized structure prediction result R482(20) for a stem-loop structure in Example 2 of the present invention.

[Fig. 14] is a graph chart showing a visualized structure prediction result R836(20) for a stem-loop structure in Example 2 of the present invention.

[Fig. 15] is a graph chart showing a visualized structure prediction result R947(20) for a stem-loop structure in Example 2 of the present invention.

[Fig. 16] is a graph chart showing a visualized structure prediction result R1338(20) for a stem-loop structure in Example 2 of the present invention.

[Fig. 17] is a graph chart showing a visualized structure prediction result R1335(20) for a stem-loop structure in Example 2 of the present invention.

[Fig. 18] is a graph chart showing a visualized structure prediction result R1587(20) for a stem-loop structure in Example 2 of the present invention.

[Fig. 19] is a graph chart showing a visualized structure prediction result R2031(20) for a stem-loop structure in Example 2 of the present invention.

[Fig. 20] is a graph showing a visualized structure prediction result R-836(20) for a recess-shaped structure in Example 2 of the present invention.

[Fig. 21] is a graph showing a visualized structure prediction result R-947(20) for a recess-shaped structure in Example 2 of the present invention.

[Explanation of Symbols]

L1      objective section of RNA
F       frame

L2        length of frame
t         constant length (shift amount of frame)
S1~Sn   small-section defined by frame

**(Detailed description of the invention)**

First, the method of the present invention will be described more concretely by using drawings.
In the present invention, "length" on RNA refers to a sequence length and means a number of sequenced bases. The smallest unit of the length is a single base, and "length" and "long" may also be read as "number of bases". Therefore, in the present specification, "having a length of 100" or the like is described, and "shift by only constant length t" means "shift by a constant number of bases t".

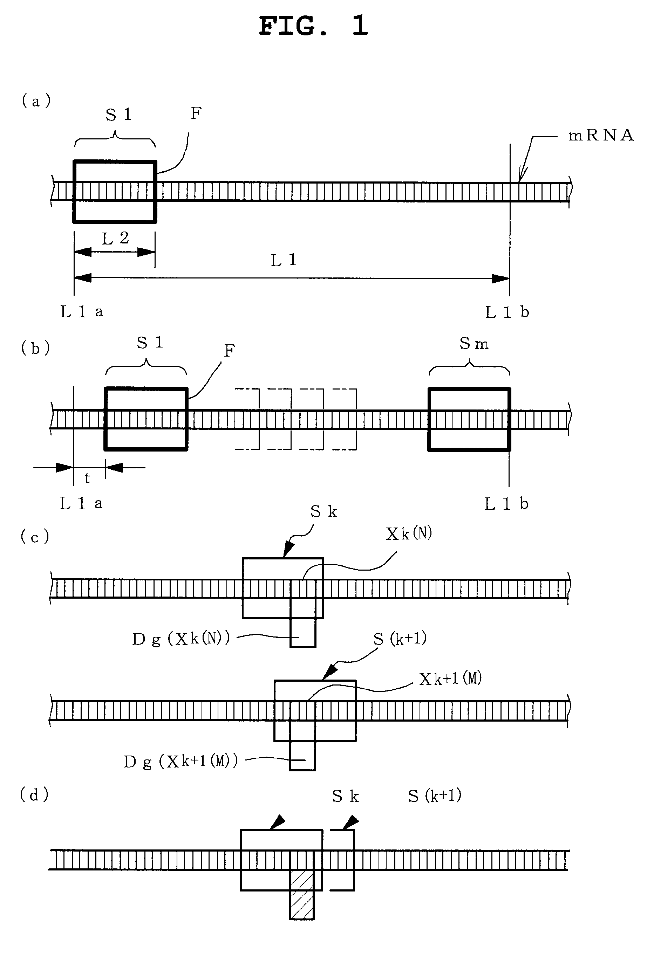

As described in the above (1), the present method includes at least the steps (A) to (C). Fig. 1 is a schematic view showing each step and principle of prediction of the method of the present invention in an easily recognizable manner. In the example of Fig. 1, mRNA is used as one example of RNA, which is represented by a band extending in the horizontal direction of the drawing. Each of the minimum sections separated by fine scale drawn in the band represents one base.

In the step of (A), first as shown in Fig. 1(a), an objective section to be predicted is selected for the entire length of mRNA. Length L1 of the objective section may be appropriately determined depending on the use, and may be an entire length of mRNA.
Here, assuming frame F having specific length L2, it is applied onto the objective section. The length L2 of the frame F should be longer than the number of bases of the specific secondary structure to be predicted.

First, as shown in Fig. 1(a), in a first small-section S1 that covers one frame from one end L1a of the objective section, a small structure that contains a specific secondary structure and is formed by mRNA is predicted. Here, the small structure is predicted by making Gibbs free energy as an index for extraction. For example, a small structure having Gibbs free energy of a predetermined value or less is extracted. Small Gibbs free energy means high possibility of presence.

As RNA which is to be an object of prediction for presence of the specific secondary structure, miRNA (used herein also including precursor RNA thereof as far as no contradiction arises), rRNA, tRNA, ncRNA and the like can be recited besides mRNA (used herein also including precursor RNA thereof as far as no contradiction arises). ncRNA is RNA that is not translated into a protein, and recently much attracts attention as functional RNA and the like.
Among these, mRNA is conventionally regarded as an important drug discovery target in development of pharmaceuticals, and there is also a finding that efficacy of antisense pharmaceuticals or siRNA depends on the secondary structure of mRNA. The finding about the secondary structure is expected to be able to contribute to elucidate a structure that is involved in a known or unknown molecular biological phenomenon such as miRNA, and on the other hand, since higher-order structure of mRNA in a living body has not been clarified at all except for some specific mRNA, mRNA is a very important object for prediction and discussion of presence of a meaningful secondary structure in RNA.
On the other hand, miRNA is generated by cutting a precursor (pre-miRNA) having a stem-loop structure with enzyme Dicer, and physically suppresses translation without cutting and degrading of mRNA. Therefore, by stabilizing the structure of the precursor RNA, it is possible to suppress generation of mature miRNA and as hence to promote translation of target mRNA. From such a viewpoint, pre-miRNA is also a preferred object for prediction.
Also, ncRNA is believed to perform some function in a living body. Certainly, it is believed that such a function be realized in a living body by RNA forming some specific higher-order structures. Further, in such ncRNAs, there are also so many ncRNAs for which whether it forms a unique structure is unclear as in rRNA, tRNA and the like. There are many cases where structure prediction for such RNA is untreatable by conventional-type structure prediction software that is unable to handle probability even though importance of such a structure is well recognized. From these viewpoints, ncRNA is also a preferred object for prediction in the present invention.

As the objective section, an arbitrary section on RNA may be selected, and it may be the entire length of the objective RNA. For example, in predicting presence of an intended secondary structure for the purpose of suppressing translation into a protein, the position predicted to greatly contribute to suppression of translation, for example, 5'-untranslated region (5'UTR), a region spanning 5'UTR and coding region (CDS), preferably a translation initiation site, the vicinity thereof and the like may be selected. On the other hand, in predicting presence of an intended secondary structure for the purpose of enhancing translation into a protein, the position predicted to greatly contribute to stability of mRNA, for example, 3'-untranslated region (3'UTR) and the like may be selected. Alternatively, for the purpose of finding a pseudoknot structure (special stem-loop structure) that causes a biological phenomenon such as frame shift, a coding region may be principally selected.
The length of the objective section is not particularly limited, however, it can be said that the longer the length, the more significant the utility of the present invention becomes. This is because unlike the conventional structure prediction software such as mfold that fails to provide a preferred prediction result for a relatively long section as described in the background art, the method of the present invention will not deteriorate the reliability of the prediction result regardless of the length of the objective section owing to its specific calculation feature, and enables discussion of the structure quantitatively from both aspects of stability and probability. More specifically, when the objective section has a number of bases of 100 or more, and particularly 200 or more, utility of the present invention over the conventional method becomes significant.

For selection of the objective section, a position of either one of ends of the objective section (in usual operation, 5'-side end) is only designated, and designation of the other end part of the objective section may not be performed by determining a frame length, constant length t and a step shifting number of times as will be described later.

A number of bases of the specific secondary structure to be determined is not particularly limited, and a lower limit and an upper limit thereof are determined by combination of the ranges taken by respective characteristic parameter values that define the specific secondary structure as will be described later. For example, when stem length is x1 to x2 bases, loop length is y1 to y2 bases, and a number of allowable mismatches in the stem-loop is i, the length of the specific secondary structure is $[x1 \times 2 + y1]$ to $[x2 \times 2 + y2 + i]$ bases. A preferred number of bases of the specific secondary structure is for example, about 10 to 100 bases, and more preferably about 20 to 60 bases.

The specific secondary structure may be the one of which the structural feature is definable by parameter values. The parameter value that defines the specific secondary structure is a characteristic parameter value.

Concrete examples of the specific secondary structure include such as a stem-loop structure, a pseudoknot structure and a recess-shaped structure.

The recess-shaped structure is a structure that is focused by the present inventor as an important secondary structure, and is a structure existing between two adjacent stem-loop structures, and having an interplace not interacting with other base sequences. Although the interplace itself is flat, a base involved in stem is present on both sides thereof, so that it is regarded as a specific secondary structure. The recess-shaped structure may also be called a groin structure.

Examples of the characteristic parameter values defining a stem-loop structure include stem length, loop length, a number of allowable mismatches in a stem-loop.

For the stem length (meaning a number of bases of either one chain contributing to formation of a base pair, and including wobbling of G-U), an upper limit may not be particularly specified, and is 4 to 50 bases, and more preferably 5 to 20 bases. The loop length is 3 to 50 bases, and more preferably 4 to 10 bases.

The number of allowable mismatches in a stem-loop is 0 to 30 bases, and more preferably 0 to 10 bases.

As the characteristic parameter values that define a pseudoknot structure, in addition to the parameters that define a stem-loop, a certain continuous number of bases that interact with a sequence on the 3'-end side in a loop part of a major stem-loop (stem-loop existing on further 5'-end side), and the distance (number of bases) of the sequence that is complementary to the part, viewed from the major stem-loop can be recited.

In the loop part, the certain continuous number of bases that interact with a sequence on the 3'-end side is preferably 3 to 50, and more preferably 5 to 20.

The distance (number of bases) of the sequence that is complementary to the part, from 3'-end of the major stem-loop is 4 to 50, and more preferably 5 to 20.

In the recess-shaped structure, stems a and b are respectively present on both sides of the centered gap part. The stems a and b on both sides individually form a base pair, and it is sufficient that numbers of inner bases of the respective base pairs of the stems a and b (that is, bases on the side of the interplace) are designated for defining the recess-shaped structure. Therefore, in the present invention, as preferred characteristic parameter values that define the recess-shaped structure, a number of bases in the centered gap part, a sum of numbers of bases of respective inner parts of stems in the two stem-loop structures on both sides of the interplace, and a number of allowable mismatches in the inner parts of the stems are employed.

The number of bases in the interplace is 0 to 50, and more preferably 0 to 10.

The sum of numbers of bases in the inner parts of the two stems on both sides of the interplace is 4 to 50, and more preferably 4 to 30.

The mismatch is 0 to 10, and more preferably 0 to 5.

A frame length may be shorter than the entire length of the objective section and longer than the aforementioned length of the specific secondary structure, and it is preferred to set the length such that a biological condition is imitated as much as possible. For example, in a process of translation of long RNA such as mRNA, translation proceeds while ribosome rearranges higher-order structure on mRNA and a protein into a single strand. That is, mRNA immediately after passage of ribosome is in a free condition capable of forming any structure. The site in such a free condition forms some higher-order structures one after another. This condition is considered to be approximate to the state assumed by an existent structure prediction technique (for example, a first calculation unit in the program of the present invention, or a known structure prediction software such as mfold). Particularly in mfold, structure prediction is conducted using experimentally obtained parameters, however, the parameters are obtained in an experiment for relatively short RNA. In other words, mfold provides a correct result only for a relatively short (empirically, 200 bases or less) sequence. Consequently, the frame length may be set so as to achieve a preferred prediction result while taking progression rate of ribosome and characteristics of such as existent structure prediction technique into account.

A concrete frame length differs depending on the kind of the specific secondary structure, however, a number of bases of the frame length in actual use is 50 to 300. When the specific secondary structure is a stem-loop structure, a number of bases of the preferred frame length is 50 to 200 from the viewpoint of reliability of the result obtainable for each frame. When the specific secondary structure is a recess-shaped structure, a number of bases of the preferred frame length is 100 to 300.

Extraction of the small structure in each small-section in the above step (A) is conducted based on Gibbs free energy, that is, the one exhibiting an energy value of not more than a predetermined value of Gibbs free energy is extracted. For this extraction, for example, RNA structure prediction software known in the art such as mfold may be used.

The characteristic parameter values of the specific secondary structure which are designated as a condition in extraction may be designated with a certain range of allowance such as "stem length a to b", "loop length c to d", and "number of mismatches e to g". As a result, the extracted specific secondary structures in the small structures include not only the completely identical ones but also similar the ones within the allowable range.

In addition, a condition of the structure prediction method itself such as mfold may be appropriately changed so as to be suitable for the purpose. For example, structure prediction is usually conducted at a temperature of 37°C, however, structure prediction of RNA found in thermophiles (bacteria growing in an environment at high temperature) is conducted at a temperature much higher than 37°C. When experimental data or the like is available, other parameters that influence on a result of the structure prediction method such as mfold or the like may be varied (for example, to designate the minimum loop length in a stem-loop, or to designate to forcibly make a certain structure incorporated).

Furthermore, in order to conduct structure analysis in a certain special condition for the RNA, parameters that influence on a result of the structure prediction method such as mfold or the like may be changed according to speculation. For example, for executing structure analysis in a condition that a protein, antisense or the like is bound to a certain region, the structure prediction method may also be used while designating that the region will not interact with any other part on assumption that a constant region containing the region does not participate in formation of structure in the RNA.

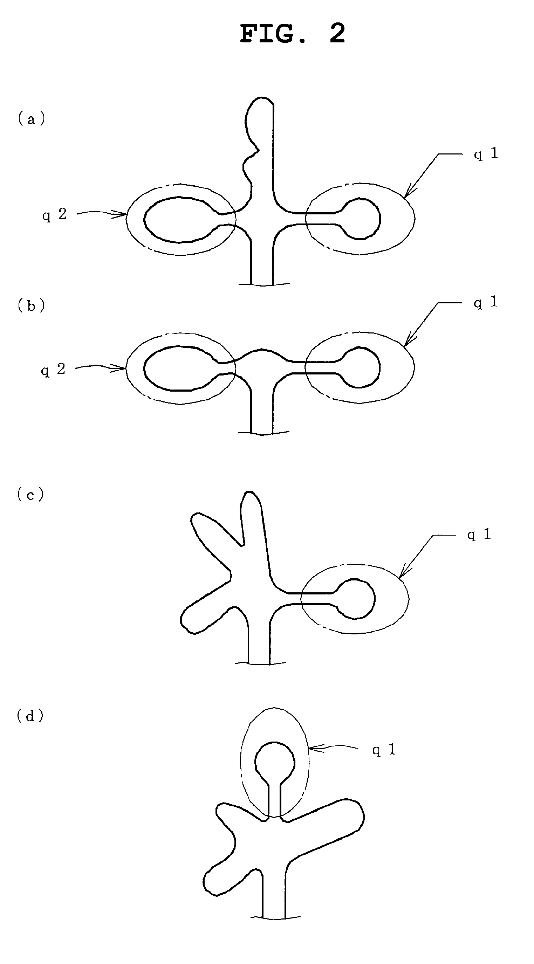

Through structure prediction in the small-section, at least one small structure is extracted from one small-section. Figs. 2(a) to (d) show examples of extraction. For ease of understanding, it is assumed that a specific secondary structure corresponding to characteristic parameters is a simple stem-loop structure, and four small structures containing the same are extracted from a single small-section. As will be described later, the method itself of extracting (predicting) a small structure may be a conventionally known technique.

For these four small structures extracted in this small-section, the position where the specific secondary structure is present is examined. In the examples of Figs. 2(a) to (d), three small structures in Fig. 2(a) to (c) have specific secondary structure q1 of the same characteristic at the identical position. A small structure in Fig. 2(d) also has specific secondary structure q1 of the same characteristic but at a position different from that in the other three structure.

Taking the position of the specific secondary structure q1 in the three small structures of Fig. 2(a) to (c) as a specific position X1(1) of the small-section S1, and what probability the specific secondary structure q1 is present in the specific position X1(1) is determined.

The simplest calculation method for this is: [(number of small structures having q1 at identical position)/(total number of small structures)]=3/4. This is made as first probability Dg(X1(1)) of the specific position X1(1) of the small-section S1. However, such a simple calculation is based on the concept that "the larger the number, the higher the probability is" and is not necessarily accurate. Even if an overwhelmingly large number of exemplifications are provided as a prediction result, it is preferable to take Gibbs free energy into account when determining the probability of a specific secondary structure being present at a specific position. A preferred example of calculation process of an index for the first probability will be described later.

This first probability provides an index for what degree of probability the specific secondary structure q1 is present in the specific position X1(1) as described in the above effect of the present invention.

On the other hand, in four small structures in Fig. 2, a small structure (Fig. 2(d)) having the specific secondary structure q1 in a different position is present as described above, and as shown in Figs. 2(a) and (b), there is also the case where a specific secondary structure q2 which is slightly different from the specific secondary structure q1 is contained in the same small structure.

In the present invention, for these, the probability of a specific secondary structure being present at a specific position is determined while making a position of the specific secondary structure as specific positions X1(2) and X1(3) of the small-section S1 in a similar manner as described above, and made as first probabilities Dg(X1(2)) and Dg(X1(3)), respectively. As described above, according to the simplest calculation method, the specific secondary structure q1 in Fig. 2(d) is Dg(X1(2))=1/4 and the specific secondary structure q2 in Fig. 2(a), (b) is Dg(X1(3))=2/4.

In this manner, what kind of the specific secondary structure is present at which position for every small structure extracted in the small-section S1 is examined, whereby what kind of the specific secondary structure is present at what probability is determined in every proved specific position. This is made as first probability Dg(X1) for each specific position X1 in the small-section S1. As described above, X1 is a value peculiar to the small-section S1 such as X1(1), X1(2) and X1(3), and first probability is given for each position.

The specific secondary structure is not necessarily extracted in a condition that it is contained in a small structure as one branch, and a prediction result may be such that only the specific secondary structure protrudes in a certain position of flat small-section.

The "Specific position" itself in the small-section is not a point (position for one base), but is a section having a base length corresponding to a specific secondary structure expanded into a straight shape. In other words, the specific position is an expanded section of the specific secondary structure which is present in a certain position in the small-section. Therefore, "at what probability the specific secondary structure is present in a specific position" can be paraphrased to "at what probability the specific position becomes a specific secondary structure".

In the present specification, description is made while calling a number given to each base for allowing identification of the position of the base in a sequence (order of sequence) "base address".

When it is convenient to represent the specific position by a base address of one point for handling in calculation or the like, the specific position may be expressed by the base address of the base at one point such as the top of the sequence section occupied by the specific position, and the base length of the specific secondary structure.

Where the specific position exists may be represented by using a relative base address indicating where it is from the top of the small-section, however, a base address indicating the position where it is from the top of the objective section may be used, or both of these may be used.

The operation for examining at what probability and at which position in each small structure the specific small structure is present, for every small structure extracted in each small-section may be conducted by using conventionally known RNA structure prediction software such as mfold.

In the above example, a value of the first probability is represented by a simple existing ratio, and in the following, a value preferred for using as the first probability will be described.

First, respective Gibbs free energy values of every small structure (Fig. 2(a) to (d)) extracted in one small-section are made as $\Delta Ga$ [kcal·mol-1], $\Delta Gb$, $\Delta Gc$, and $\Delta Gd$.

In such a case, for the specific secondary structure q1, a value of the first probability to be given to the specific position is calculated by the formula:

$$Dg=[(\Delta Ga+\Delta Gb+\Delta Gc-\Delta Gd)/4].$$

The value obtained by the above formula is a preferred index for quantitatively evaluating provability in which the specific secondary structure q1 is present at a certain specific position proved in one small-section.

Also "%Dg" that is a value calculated by dividing a value of Dg obtained from the above formula by a value of $\Delta G$ of the most stable structure (structure having the smallest Gibbs free energy $\Delta G$) of the small structure extracted in the small-section, followed by multiplication by 100 may be used.

Theoretically, the existing ratio at that temperature can be calculated insofar as $\Delta G$ value of each small structure predicted for each small-section is available (on assumption that each small structure is in equilibrium). The existing ratio calculated in such a manner may also be used as the aforementioned "%Dg" (or a value obtained by dividing this value by 100, and multiplying by $\Delta G$ of the most stable structure (structure having the smallest Gibbs free energy $\Delta G$) may be used as "Dg"). However, since $\Delta G$ of each predicted small structure is merely a predicted value in this calculation, likelihood of presence is discussed mainly by using the first probability.

Next, frame F is shifted toward the other end side by constant length t (t<L2) as shown in Fig. 1(b), and a small-section covered by the frame F at this time is made as S2.

The constant length t which is a shift amount of frame may be a value shorter than length L2 of the frame so that the small-sections overlap with each other, and by increasing the number of overlapping, reliability of the value of probability itself can be improved. The constant length t is preferably a small value around one base, however, it may be set at an appropriate value depending on such as performance of calculation unit being used, degree of required reliability, and biological environment in which RNA which is an object of calculation is placed because the number of the small-sections to be subjected to calculation increase with the constant length. From these points, a preferred range of a number of bases of the constant length t is 1 to 10, and particularly 1 to 3.

Similarly to the operation (or analysis) in the small-section S1 as described above, a small structure is extracted also in the small-section S2, and the specific position where the specific secondary structure is present is examined, and the first probability Dg(X2) for each specific position in the small-sectionS2 is obtained.

In this manner, an operation of determining the first probability for each specific position in the small structure that is extracted in the small-section when the frame F is shifted toward the other end by the constant length t is repeated up to the last small-section Sn when the frame F reaches other end L1b of the objective section as shown in Fig. 1(b).

Through repetition of the above operation, first probabilities Dg(X1) to Dg(Xn) for each specific position in each small-section are obtained. The subscript n for Sn and Xn, and the subscript k for later-described Sk represent an integer indicating the number of small-section.

In the above step (A), description is made so that the objective section is analyzed sequentially from the end while a frame is shifted sequentially, however, it does not only necessarily mean that the calculation and operation may be performed from the end in the order. In other words, the first probability may be determined while randomly choosing the small-sections S1 to Sn that partly overlap with each other from the objective section. That is, the above step (A) means obtaining a condition set and analyzed for each of small-sections overlapping with each other in the objective section. In progression of calculation and program, sequential analysis from end is a preferred procedure.

Two adjacent small-sections overlap with each other by (L2-t) due to fine shifting of frame F. By setting t to be very small relative to L2, a base part of a certain length (the part becomes a specific secondary structure) in one small-section is also contained in other small-sections overlapping with it, and it is rendered an object for structure prediction peculiar to the small-section in the base sequence of each small-section.

As described above, any of the first probabilities Dg(X1) to Dg(Xn) for each specific position in each small-section determined herein are values given to at least one specific position in each small-section.

As shown in Fig. 1(c), the first probability Dg(Xk(N)) is given to a certain specific position [Xk(N)] in a certain small-section Sk, and the first probability Dg(Xk+1(M)) is given to a specific position [Xk+1(M)] in a neighboring small-section Sk+1. In Fig. 1(c), magnitude of each first probability is represented by the length of downward bar graph.

These operations form the step (A).

In the step of (B), as is already described in the above description of effect of the present invention, each specific position (first probability is given to each position) in each small-section proved through the operations of step (A) is applied to a corresponding position on the objective section. Since each small-section is defined so that they overlap with each other, specific positions overlapping with each other arise in each small-section as shown in Fig. 1(d) In the example of Fig. 1(d), a certain specific position in a small-section Sk and a certain specific position in the S(k+1) coincide and overlap with each other. The degree of coincidence and overlap between these specific positions is various, and sometimes they are isolate and do not overlap with each other, or sometimes they overlap by the maximum possible number for each small-section. Here, the degree of overlapping between specific positions is determined and called second probability.

This second probability may be represented by a number of overlapping degree, or may be represented by a numerical value calculated by dividing degree of overlapping by a total number of small-sections in which the entire sequence of the specific secondary structure is included, followed by multiplication by 100.

In the step of (C), based on the first probability and the second probability determined in the steps (A) and (B), presence of the specific secondary structure in the objective section is predicted. For example, it can be predicted that the one in which both first probability and second probability are high is a specific secondary structure having high possibility of actual presence.

In order to indicate where the specific position exhibiting high first probability and second probability is present more effectively to a person practicing the method, it is preferred not only to display numerical values of these two probabilities simply side by side but also to display them visibly in one graph while two probabilities are correlated with each other. A concrete method of displaying will be exemplified in description of program as will be described later.

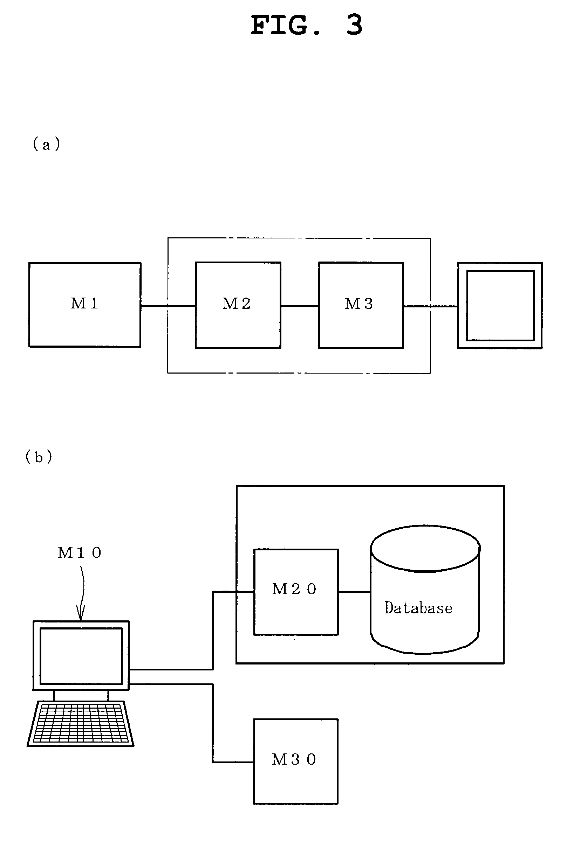

Next, configuration of an apparatus for practicing the method of the present invention will be concretely shown. Fig. 3 is a block diagram schematically showing one exemplary configuration of the present apparatus. As shown in the above (13) and in Fig. 3, the apparatus includes at least an input unit M1, a first calculation unit M2, a second calculation unitM3, and an output unit M4.

The input unit M1 is an apparatus unit for inputting initial values required for prediction, such as primary structure data of RNA (mRMA) which is an object of prediction, an objective section to be an objective of prediction for the primary structure, characteristic parameter values of a specific secondary structure, a frame length, constant length t and the like. The primary structure data is not necessarily data of the entire RNA (mRMA), and part of the primary structure data may be inputted according to the objective section to be an object of prediction.

These initial values are as described in the method of the present invention.

The first calculation unit M2 is an apparatus unit that executes the step (A) of the method of the present invention as described above, and defines small-sections S1 to Sn while finely shifting the frame in the objective section, and executes calculation for determining first probabilities Dg(X1) to Dg(Xn) for each specific position in each small-section. The second calculation unit M3 is an apparatus unit that executes the step (B) of the method of the present invention as described above, and executes a calculation that determines at which position and to what extent on the objective section the specific positions proved in respective small-sections overlap with each other and makes it as second probability.

The process of calculation for determining the first probabilities Dg(X1) to Dg(Xn) executed in the first calculation unit M2, and the process of calculation for determining the second probabilities executed in the second calculation unit M3 are as described in the method of the present invention.

The output unit M4 may be various image display devices such as a liquid crystal display monitor, or various print output devices such as a printer, and each output device may be associated with a special computer.

As shown in Fig. 3, in a preferred mode of the apparatus, the first calculation unit M2 and the second calculation unit M3 are configured as an integrated or independent computer(s). In this case, the input unit M1, and the output unit M4 are respectively an input device and an output device associated with the computer, however, the input device may be a computer for a client.

Example shown in Fig. 3(a) is an exemplary configuration in which the present apparatus is implemented by a single computer. Required primary structure data of RNA may be supplied by access to CD-ROM or external database (external computer) in response to an instruction by an operator, as well as being stored in its own storage unit. As an example of such primary structure data, "GHGD_transcripts_R27f.fasta" provided from Celera Genomics, "nuc_all.fa" provided from H-invitational or the like can be recited.

In the example of Fig. 3(b), functions of the input unit M1 and output unit M4 are given to a computer M10 of a client. The client designates a name of required primary structure data of RNA, and an objective section, and inputs required initial values such as characteristic parameter values of a specific secondary structure, length L3 of a frame and constant length t, and is able to watch the result on the monitor. Database for storing the primary structure data of RNA is managed by an external computer (such as server) M20. Main calculation functions of the first calculation unit M2 and the second calculation unit M3 are given to another external computer M30 to allow appropriate use of a plurality of structure prediction software. These computers are connected via a communication line such as LAN or the INTERNET.

Next, a configuration of a program for practicing the method of the present invention will be concretely shown. The program is a program that is configured to make a computer execute the method of the present invention when the apparatus of the present invention is configured by the computer. The program may be a mode which is executed in a single computer, as well as may be a mode which is executed in a plurality of computers in such a manner that a main program is placed in a computer at hand, and initial values are sent to a calculation program in other computers, and a calculation result is returned to the main program, as shown in Fig. 3(b).

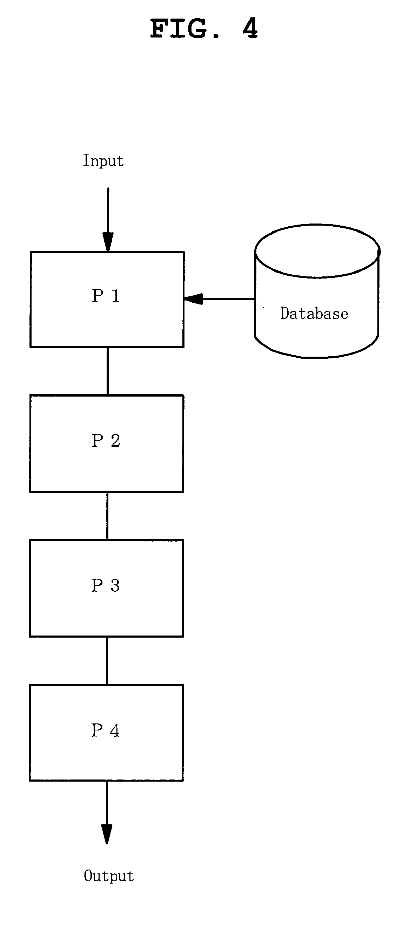

As shown in the flow of Fig. 4, the program is basically the same with the flow of the method of the present invention, and includes Step P1 for making a computer function as an input means, Step P2 for making a computer function as a first calculation means, Step P3 for making a computer function as a second calculation means, and Step P4 for making a computer function as an output means that displays the respective numerical values of the first probability and the second probability on an output device to allow browsing.

In the Step P1, necessary initial data such as primary structure data of RNA which is an object of prediction, an objective section, characteristic parameter values of a specific secondary structure, a frame length and constant length t is retrieved. These initial values are as described in the above method of the present invention.

A program may be configured so that the characteristic parameter values of a specific secondary structure, a frame length, constant length t and the like are directly inputted by an operator, or may be configured so that appropriate values are displayed to allow an operator to select.

A program may be configured so that the primary structure data of RNA is retrieved by accessing to its own storage device (HDD, CD-ROM and the like) or external database based on a data name inputted by an operator.

The Step P2 is a program part that executes a calculation part of the step (A) in the method of the present invention as describe above, defines the small-sections S1 to Sn by shifting the frame in the objective section, and conducts calculation for determining the first probabilities Dg(X1) to Dg(Xn) for each specific position in each small-section. For prediction of the small structure in each small-section, it may be configured so that a known structure prediction program such as mfold can be used as a sub routine by linking with an external computer.

The Step P3 is a program part that executes the step (B) of the method of the present invention as described above, and executes a calculation that determines at which position and to what extent on the objective section the specific positions proved in each small-section overlap with each other and makes it as second probability.

The process of calculation for determining the first probabilities Dg(X1) to Dg(Xn) executed in the Step P2, and the process of calculation for determining the second probabilities executed in the Step P3 are respectively as described in the method of the present invention.

The Step P4 is a program step that outputs the value of the first probability and the value of the second probability determined in the above Steps P2 and P3 in forms of available forms.

The first and second probabilities may be outputted in such a manner that they are displayed so that a user can easily review both values. For example, in one mode, the respective values of the first and second probabilities may be outputted in parallel, in a table, simply in correspondence with the objective section, and for each value, a value exceeding a specified value is marked, while in another mode, a criterion may be provided for each of the first and second probabilities, and a specific position that satisfies both criteria may be picked up and displayed.

In a preferred display mode, an output may be made in two- or three-dimensional graphic design in which the first and second probabilities are concurrently shown in a graph where one axis corresponds to the objective section so that the specific position exhibiting high first and second probabilities can be intuitively recognized. An example of such a display will be shown next.

The present example is an example in which the first probability is shown in a format of a bar graph in a graph where one axis corresponds to the objective section, and the second probability is also shown concurrently.

For example, in calculation of the Step P2, when first probability $Dg(Xk(N))$ is given to a certain specific position $[Xk(N)]$ in a certain small-section $Sk$, and first probability $Dg(Xk+1(M))$ is given to a specific position $[Xk+1(M)]$ in an adjacent small-section $Sk+1$, a bar (graph bar) of a bar graph is displayed at each corresponding specific position on the objective section in the graph as shown in Fig. 1(c), and magnitude of each first probability is represented by length of the graph bar.

The values of the first probability are drawn as graph bars while making at least one specific position obtained in each of the small-sections S1 to Sn correspond to the objective section of the graph, and then a large number of overlaps between specific positions (=specific secondary structures) arise and graph bars also overlap with each other depending on the position as shown in Fig. 1(d).

Then as shown in Fig. 1(d), when the degree of overlapping between graph bars is high, or when the second probability is high, the display is made while changing colors and/or patterns of the overlapping parts.

To change the color, an arbitrarily factor selected from hue (corresponding to wavelength of monochromatic light), chroma (clearness, that is the degree of not being whitened), luminosity (brightness, that is strong and weak of light) may be changed. For example, various devises may be made in display for allowing a viewer to easily recognize, such as by displaying a graph bar having higher second probability in deep color, changing a color of the graph bar from blue to red, or making the most important part blink on the monitor screen. In the case of a monochrome display apparatus, there may be a case where various patterns such as hatching or half-tone dot meshing may be useful for recognition.

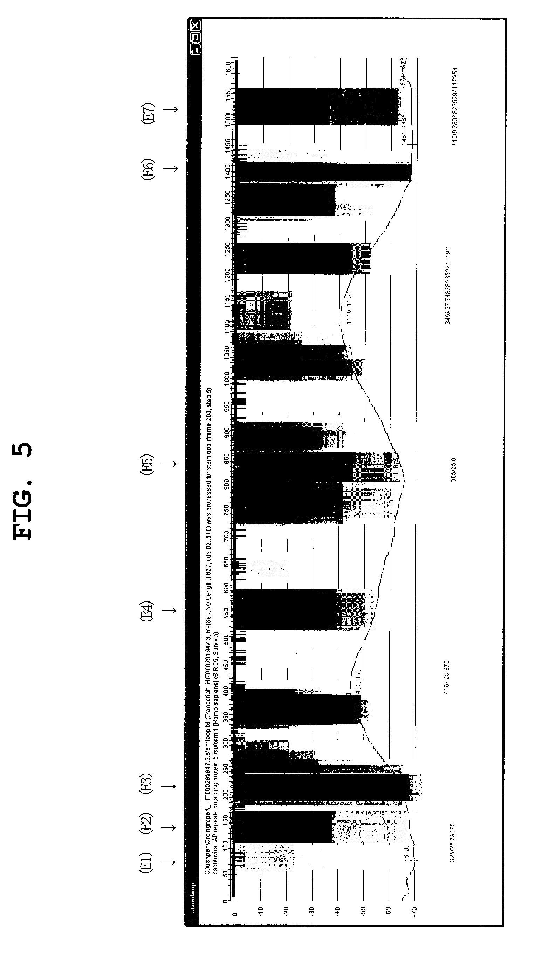

By displaying in this manner, the first probability is intuitively recognized according to the length of the graph bar, and the second probability is intuitively recognized according to a difference in color or pattern of the graph bar. For example, in the example of Fig. 5, the longer and deeper graph bar represents a specific position to be determined as a prediction result.

Fig. 5 is an exemplary display of actual analysis results in which values of the first and second probabilities are concurrently displayed in one graph over the entire objective section. In the graph, the objective section corresponds to the horizontal axis, and the value of the first probability corresponds to the vertical axis. The second probability is represented by deepness of the graph bar.

As is apparent from the bar graph of Fig. 5, the display is configured to allow a user to intuitively recognize where the specific position (=specific secondary structure) exhibiting high first probability and second probability is present according to a length of the graph bar and deepness of the graph bar.

The step that displays such a prediction result may be an independent program specialized for display which is separate from the calculation step.

As an option for display, for example, a threshold of the second probability (overlapping degree between graph bars) is made changeable, and the specific secondary structure not having overlapping degree of the threshold or higher may not be displayed. As a result, a structure of pale color is no longer displayed.

Further, a threshold of the first probability is made changeable, and a structure exhibiting first probability that is lower than the threshold may not be displayed. When the first probability is represented by function $f(\Delta G)$ using $\Delta G$ of each small structure, there is a case where the smaller the value of function $f(\Delta G)$, the higher the first probability is meant. In such a case, a structure having $f(\Delta G)$ higher than the threshold is made not to be displayed. Through these operations, it is possible to prevent the specific secondary structure having low likelihood of presence from being taken into account.

As shown in Fig. 5, a method of displaying a prediction result of the specific secondary structure in a form of spectrum is a characteristic display method where utility of the present invention is most significant. However, there is a case where it is unclear that the part displayed by a long graph bar and sufficiently overlapping is derived from the overlap between the identical specific secondary structures or is extraction results of different specific secondary structures that happened to be displayed in overlapped manner.

For improving the reliability of prediction, it is necessary to clarify that the part where graph bars are displayed in greatly overlapped manner results from the overlap of identical specific secondary structures. In the following, two approaches for this clarification will be proposed.

One approach is useful for a case where a specific secondary structure is a structure having a stem such as a stem-loop structure or a recess-shaped structure, and a state of a base is examined for the specific structures in all specific positions superimposed on the graph, and the following two kinds of bases (i), (ii) are displayed in an overlapped manner so that they can be discriminated from each other (for example, in different color).

(i) a base that always fails to form a base pair (stem) (called "free base")

(ii) a base that fails to form a base pair in a certain specific secondary structure, but participates in formation of a base pair in other specific secondary structures (called "pseudo-free base")

For example, when the specific secondary structure is the simplest stem-loop structure, and only specific secondary structures having the same structure are overlapped with each other and high second probability is displayed, it is highly possible that only a free base (corresponding to a loop part) is displayed in the center of the specific position and a pseudo free base is not displayed (exception to be noted will be described later). In this manner, reference to the free base and the pseudo-free base will be an index for determining whether the height of the second probability is derived from overlapping of the identical specific secondary structures.

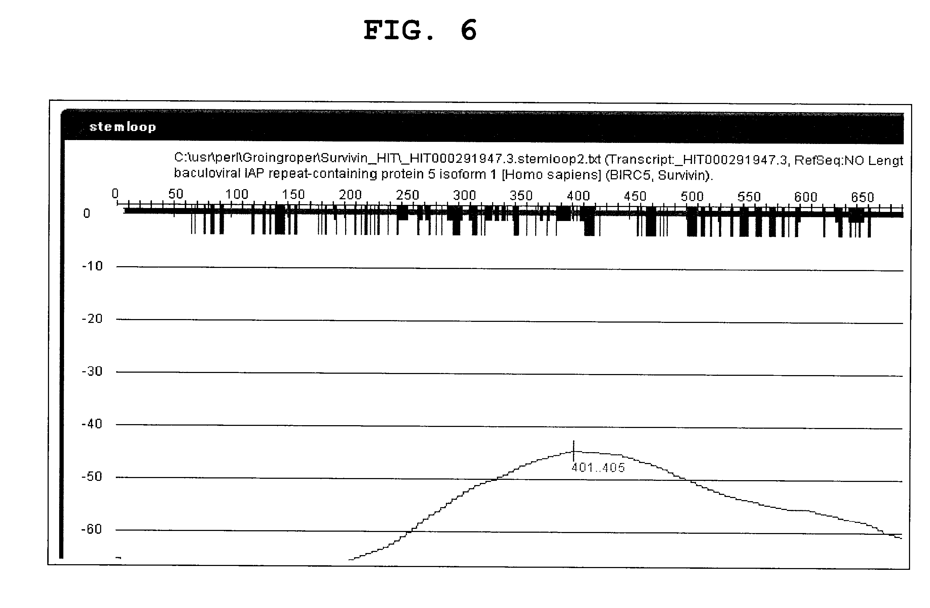

In Fig. 5, a small bar graph indicating presence or absence of the free base and the pseudo-free base is displayed at the root of the main bar graph representing the first probability. Fig. 6 is a partially enlarged view of the small bar graph indicating presence or absence of the free base and the pseudo-free base. In an actually output graph, each small bar graph is displayed in different colors so as to be clearly discriminated, however, in the drawing of the present application, it is shown in black and white shading. A higher and light bar graph represents the free base, and a lower and dark bar graph represents the pseudo-free base.

Another approach is a method in which stability value ΔG of the specific secondary structure itself is calculated, for example, by subjecting sequence of the specific position displayed in a graph to structure prediction again, and the resultant ΔG is superimposed on the graph.

When a height of the second probability is derived from overlapping of the identical specific secondary structures, ΔG value of the overlapping structures is identical, whereas when the bar graph is derived from a plurality of structures, it is highly possible that ΔG values of the overlapping structures are different from each other.

In practicing the method of the present invention, attention should be paid in the following two points.

One point is that the second probability is low in both ends of the objective section (degree of overlapping is reduced). This is merely attributed to reduction in number of overlapping small-sections on both ends. Therefore, in discussing about a secondary structure near end of the objective section, it may be finely examined whether lowness of the second probability (for example, lightness of color of the graph bar) results from a small number of overlapping small-sections, or from lowness of secondary probability of that structure.

The other point is that even if a pseudo-free base is observed as a result of reference to a free base and a pseudo-free base, not all of the specific secondary structures in that position exhibit low probability of presence. For example, now a specific secondary structure X which is present at sufficient likelihood is predicted for one sequence. However, there is a case where the secondary structure X cannot be formed in a frame not containing the entire length of the sequence forming the secondary structure X with relation to the frame, and another secondary structure Y can be predicted instead in such a manner that the sequence participates in the structure. Also in such a case, as a final output result, overlapping of different structures is outputted, and the pseudo-free bases are outputted for these two structures. However, in this case, it is obvious that probability of the specific secondary structure X is higher. For such Y, it can be removed in most cases by setting a threshold for the first or second probability as described above, however, it may strictly examine the details.

**Examples**

Example 1

In the present example, for mRNA(HIT000291947) encoding Survivin, secondary structure prediction was actually conducted for the entire section.

As for an apparatus configuration, a program including the Steps P1 to P4 as shown in Fig. 4 was created to make the method of the present invention practicable, and executed on a single computer shown in Fig. 3(a).

For prediction of the small structure in Step P2, mfold was used.

Sequence information of mRNA was obtained from H-Invitational Database.

A feature of the specific secondary structure is made as a stem-loop structure, and stem length: 10 or more bases, loop length: 10 or less bases, and a number of allowable mismatches in stem-loop: 20 bases (including number of bases in loop part) were inputted as characteristic parameter values defining the structure.

The frame length was made 200 bases, and constant value t which is a shifting amount was made 5 bases.

Prediction result is outputted in a form of one graph in which the horizontal axis represents an objective section and first probability is represented by length of the graph bar, and second probability is represented by darkness of color of the graph bar. The graph is shown in Fig. 5.

Further, for the entire objective section, a free base and a pseudo-free base are superimposed on the graph of Fig. 5 to allow further focusing on prediction.

From the obtained graph of Fig. 5, the one having long length and dark color of the graph bar was selected. At this time, as for a graph bar near end of the objective section, it was selected if the graph bar was long because its color was light.

In the primary selection, seven positions from (E1) to (E7) can be recited. Even at this point of time, sufficient focusing is achieved on prediction, and the object of the present invention is achieved, however, further focusing on a prediction result was made from the viewpoint of absence of a pseudo-free base. As a result, as for the respective specific secondary structures (motif) in positions specified by (A) base address 60 to 107, (B) base address 110 to 173, (D) base address 531 to 595, and (G) base address 1499 to 1571, since the pseudo-free base is not observed, it can be estimated that they are stable (first probability is high) and only identical motifs are abundantly overlapped (second probability is truly high). Slightly light color of (A) (second probability is low) is merely because the motif is positioned near end of the objective section.

Among these four motifs, the motif of (A) is identical to motif Suv SL1 for which ability to form a relatively stable stem-loop structure is experimentally proved in Patent document 1 described above. This suggests that the method of the present invention enables local secondary structure prediction of RNA which can be actually present in a living body, and demonstrates high reliability of the method of the present invention.

Example 2

Patent document 2(WO 2004/011610 A2) discloses an antisense chain effective for polo-like kinase 1(plk1). In the present example, why the antisense chain described in Patent document 2 is effective, and what biological phenomenon influences on activity of the antisense chain will be additionally interpreted by conducting RNA structure prediction using the method of the present invention.

Since the antisense chain described in Patent document 2 is considered to act on a known transcript plkl, first, structure prediction by the present invention was conducted on sequence data registered in GenBank under the accession No. NM_005030.

For allowing direct reference to the base numbers described in Patent document 2, in the sequence disclosed in NM_005030, only sequence part having 2190 bases shown in SEQ ID NO: 4 in the sequence list in Patent document 2 was used.

As for the structure prediction, prediction about a stem-loop structure and prediction about a recess-shaped structure were separately conducted.

As for the characteristic parameter values about the stem-loop structure, loop length was made 20 or less, stem length was made 10 or more, and stem mismatch (including loop length) was made 30 or less.

As for the characteristic parameters about the recess-shaped structure, a number of bases in an interplace of the recess-shaped structure was made from 1 to 30, a sum of numbers of bases of inner parts of the respective stems in the stem-loop structures on both sides of the interplace was made 10 or more, and allowable mismatch was made 4.

Also in any case, a number of bases of the entire length of frame L2 necessary for practicing the method of the present invention was made 200, and a number of bases of constant length t which is a shifting amount was made 3.

These parameter values are completely the same in other structure predictions conducted below.

The structure prediction result for the stem-loop structure executed according to the method of the present invention is named "R", and the structure prediction result for the recess-shaped structure is named "R-", and these prediction results are respectively visualized and outputted as a graph similarly in Example 1. In the graph, the horizontal axis represents an objective section, first probability is represented by length of the graph bar, and second probability is represented by darkness of color of the graph bar. Any graphs indicating structure prediction results below are represented in the same display format.

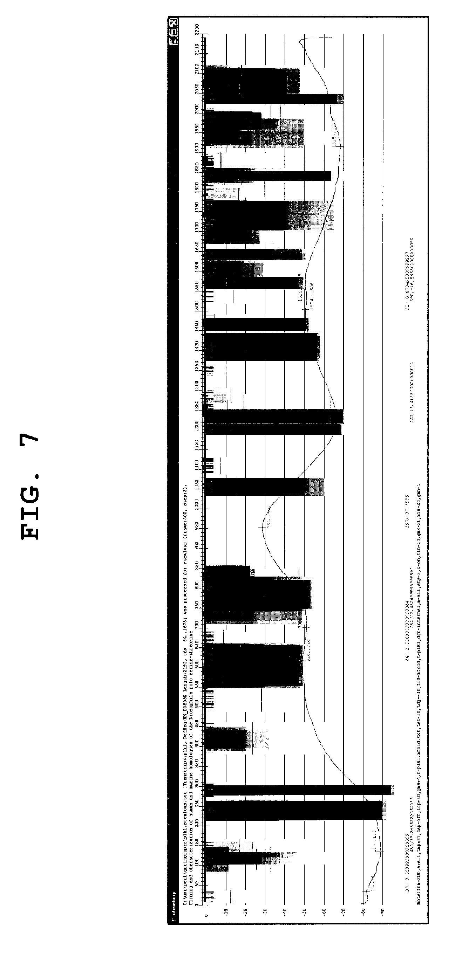

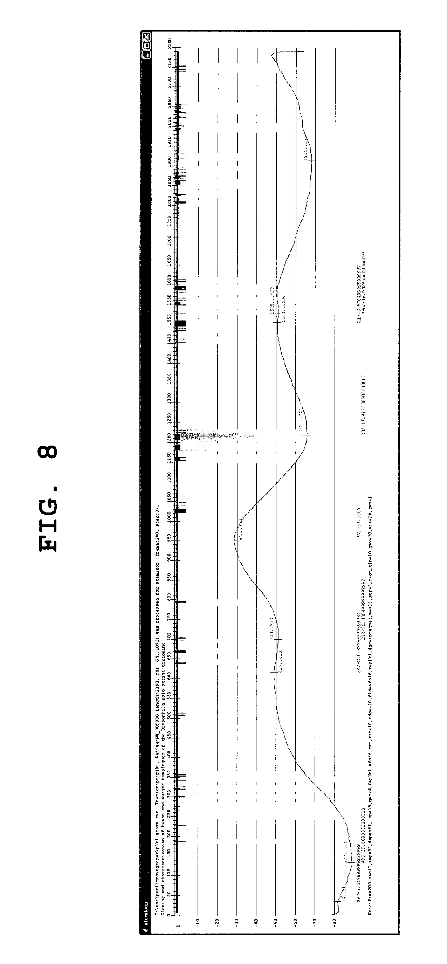

A graph visualizing the structure prediction result R is shown in Fig. 7, and a graph visualizing the structure prediction result R- is shown in Fig. 8.

Next, assuming that a site to which an antisense chain binds does not interact with NM_005030 itself, the first probability and the second probability were calculated. For prediction of the small structure of each section, mfold was used.

Since a site to which an antisense chain binds forms a double-stranded chain with the antisense chain by that binding, and is not involved in formation of a complementary strand in a molecule, the site is in a form of a single strand when only the molecule is taken into consideration. By assuming that the site to which an antisense chain binds does not interact with NM_005030 itself, energy for a transcript to form a structure suitable for binding is first discussed. Energy for interaction between the antisense chain and the transcript will be discussed later.

(i) First, a structure when antisense chains (having base sequences respectively shown in SEQ NOs: 56, 74 and 77 in a sequence list of Patent document 2) having an inhibitory activity of 75% or higher bind was predicted. That is, since these antisense chains have a length of 20 bases, and addresses of leading bases of the respective binding sites are 1144, 1814 and 1865, it was designated that these sections are in a single strand on assumption that these antisense chains are bound. For example, when a complementary antisense chain binds to a section of 20 bases from the position of base address 1144, structure prediction was conducted while designating the corresponding region such as "-PREVent1 =1144,0,20". In other words, this structure prediction can be said to be structure prediction conducted while forcibly designating a state that a binding site is kept empty as a single strand so that the transcript can bind with the antisense chain, by inputting of parameters.

Among the input example "1144,0,20", the second numerical value "0" is a number when a single strand is forcibly designated in mfold used in the present invention. More specifically, this second numerical value is a parameter for designating a stem length allowable in [the section designated by first and third numerical values (for example, section of 20 bases from base address 1144)], however, in the case of a single strand, it is 0 because presence of the stem is not allowed.

(i-a) Structure prediction was conducted for each stem-loop structure when antisense chains (addresses of leading bases: 1144, 1814 and 1865, a length of 20 bases) exhibiting an inhibitory activity of 75% or higher bind. Numerical value sets obtained as a result of each prediction were named R1144(75), R1814(75), and R1865(75), respectively. Numerical value following R is an address of a leading base at which the antisense chain binds.

R1144(75), R1814(75) and R1865(75) which are individual results of the structure prediction are numerical value sets obtainable by the prediction method of the present invention, and include a set of numerical values of the first probability calculated for each specific structure of the specific position of each frame, and a set of numerical values of the second probability representing the degree of overlapping of these on the objective section.

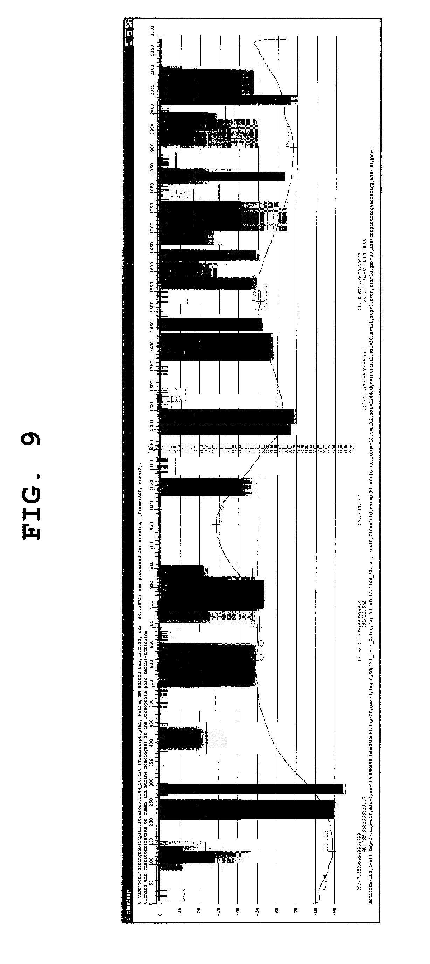

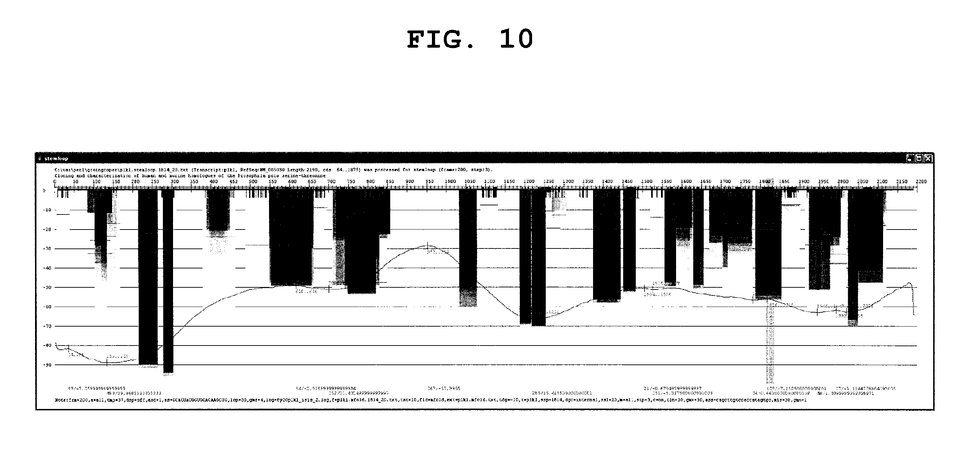

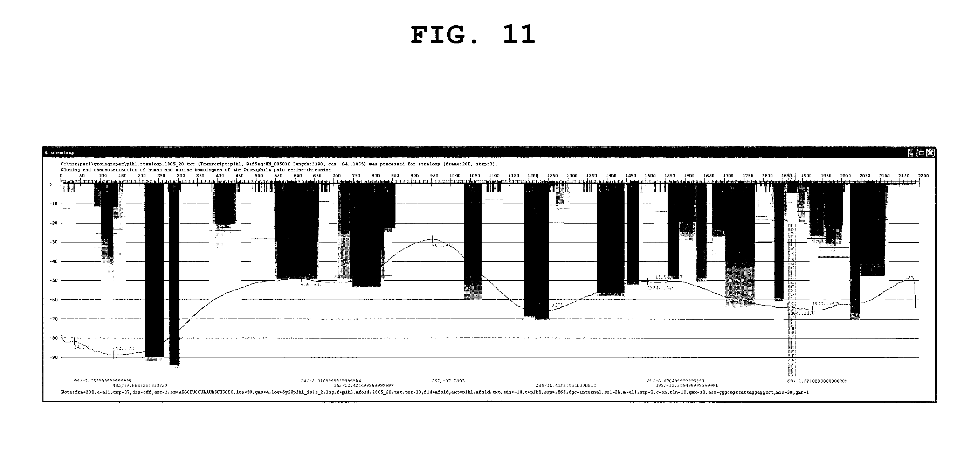

Fig. 9, Fig. 10 and Fig. 11 show bar graphs respectively showing R1144(75), R1814(75) and R1865(75) visibly. In the drawings, the region where a antisense chain binds is indicated by half-tone dot meshing as a band region vertically traversing the graph. The width of the band region is the width of a region where an antisense chain binds (the same applies to other graphs in the present example).

As is apparent from the graphs of Fig. 9 to Fig. 11, the first probability of a binding site of an antisense chain is zero only in the graph (R1144(75)) of Fig. 9, and a prediction result that a stem-loop structure cannot be formed in the section between base address 1144 and 1163 was obtained.

(i-b) Next, structure prediction was conducted for each recess-shaped structure when the above antisense chains (addresses of leading bases: 1144, 1814 and 1865, a length of 20 bases) exhibiting an inhibitory activity of 75% or higher bind, in a similar manner as the foregoing structure prediction for the stem-loop structure, and the respective prediction results were named R-1144(75), R-1814(75), and R-1865(75).

These prediction results are visibly shown by bar graphs, and no graphs include such a result that the first probability of binding site of the antisense chain is zero.

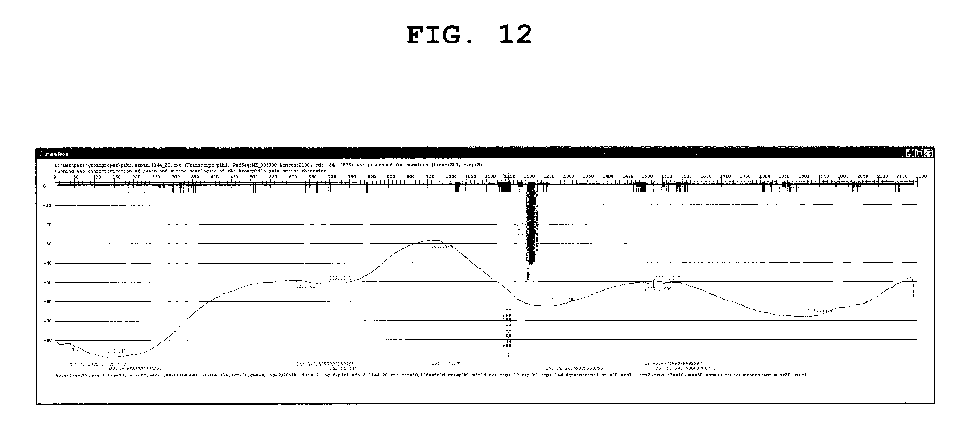

As a representative example of these, a bar graph visibly showing R-1144(75) is shown in Fig. 12.

(ii) In a similar procedure as in the above (i), the structure when antisense chains having an inhibitory activity of 20% or less bind was predicted. Addresses of leading bases of binding sections are 482, 836, 947, 1338, 1355, 1587 and 2031, and their lengths are 20 bases. Assuming that antisense chains bind in these sections, a designation was made so that these sections are in a form of a single strand.

The process of calculation in structure prediction is similar to that in the case of the above antisense chains having an inhibitory activity of 75% or higher.

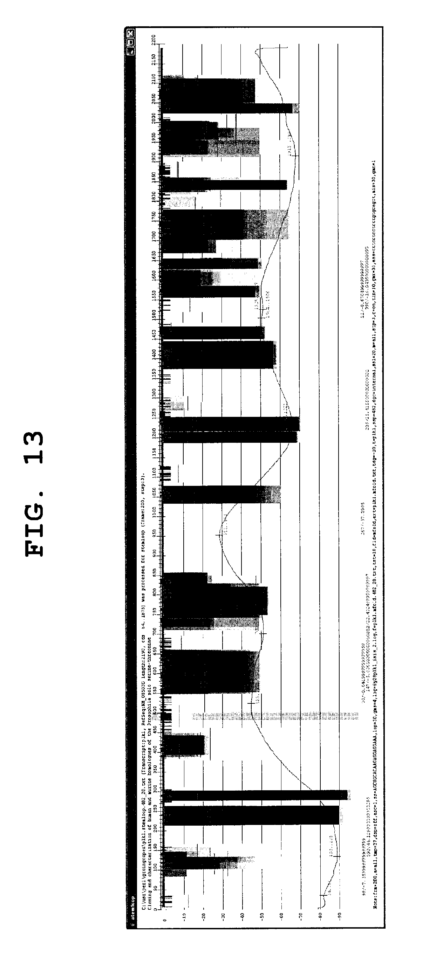

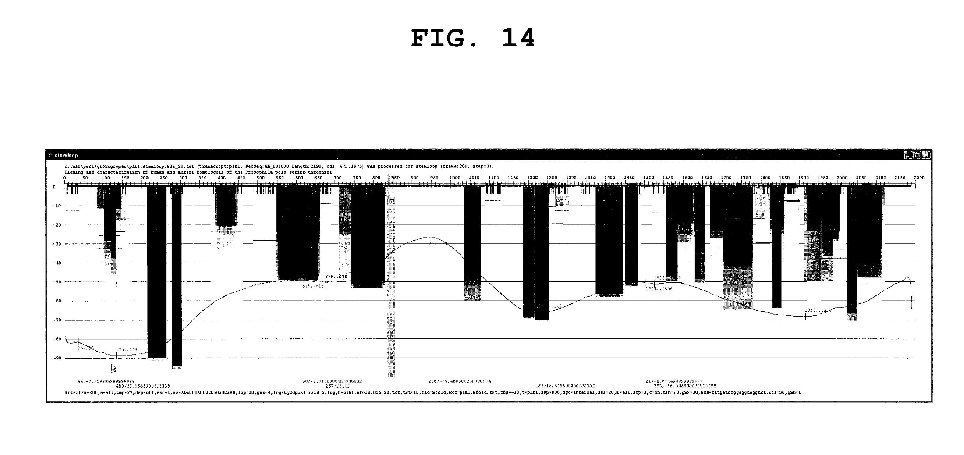

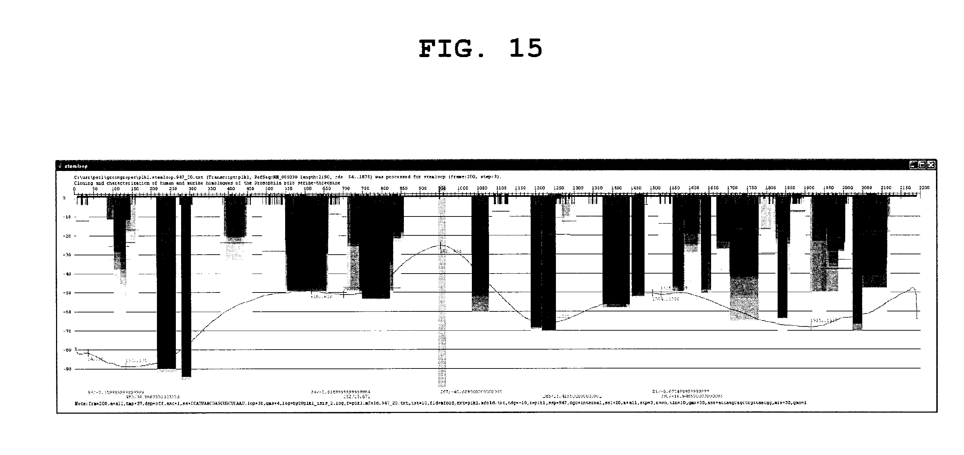

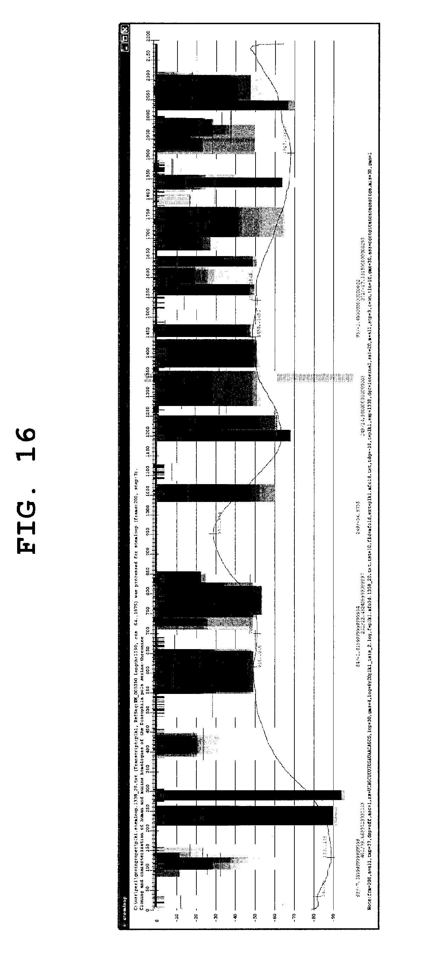

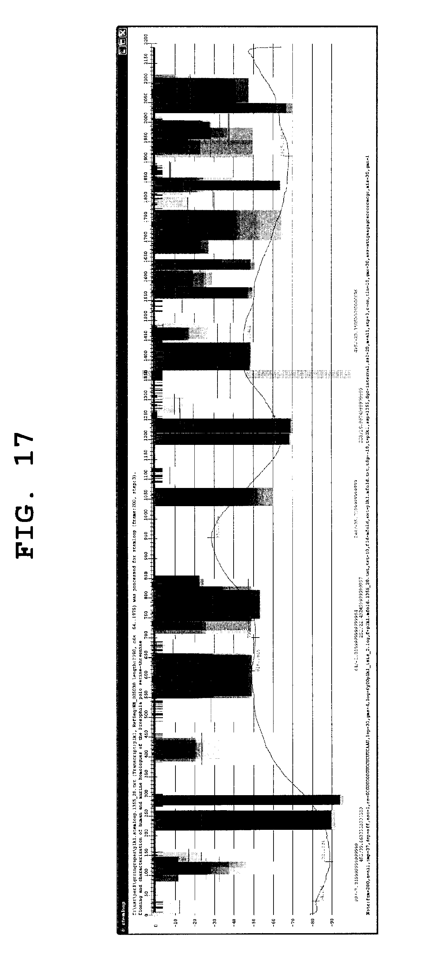

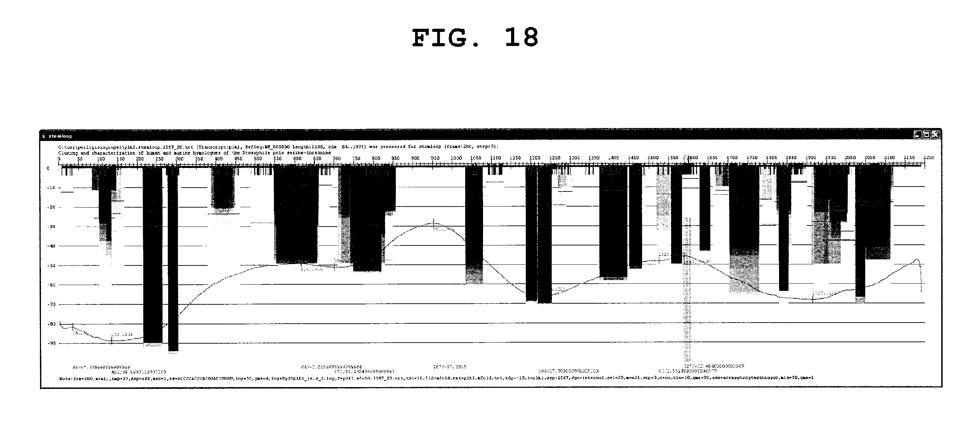

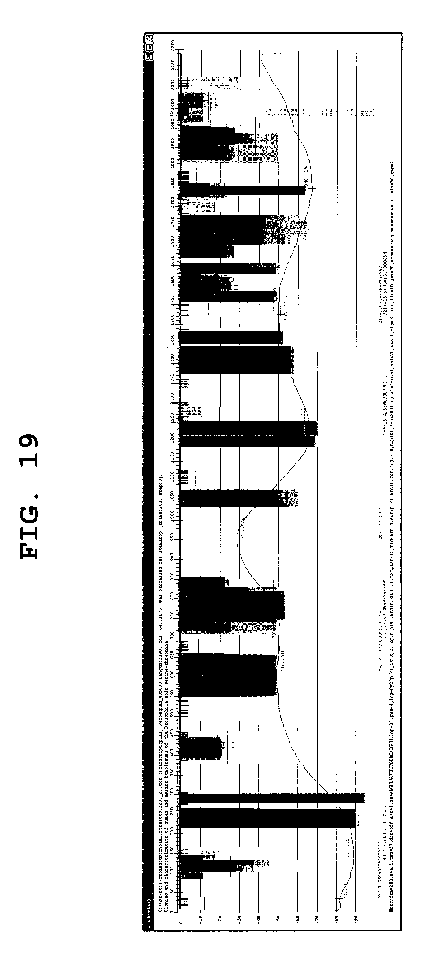

(ii-a) Structure prediction was conducted for each stem-loop structure when antisense chains exhibiting an inhibitory activity of 20% or less bind to the above binding sections. Numerical value sets obtained as a result of each prediction were named R482(20), R836(20), R947(20), R1338(20), R1355(20), R1587(20) and R2031(20), respectively. Fig. 13 to Fig. 19 show bar graphs visibly showing these prediction results.

(ii-b) Structure prediction was conducted for each recess-shaped structure when antisense chains exhibiting an inhibitory activity of 20% or less bind to the above binding sections. Numerical value sets obtained as a result of each prediction were named R-482(20), R-836(20), R-947(20), R-1338(20), R-1355(20), R-1587(20) and R-2031(20), respectively.

These prediction results are visibly shown in bar graphs, and the first probability of binding site of the antisense chain was zero only for R-947(20), and a prediction result that a recess-shaped structure cannot be formed in this section was obtained.

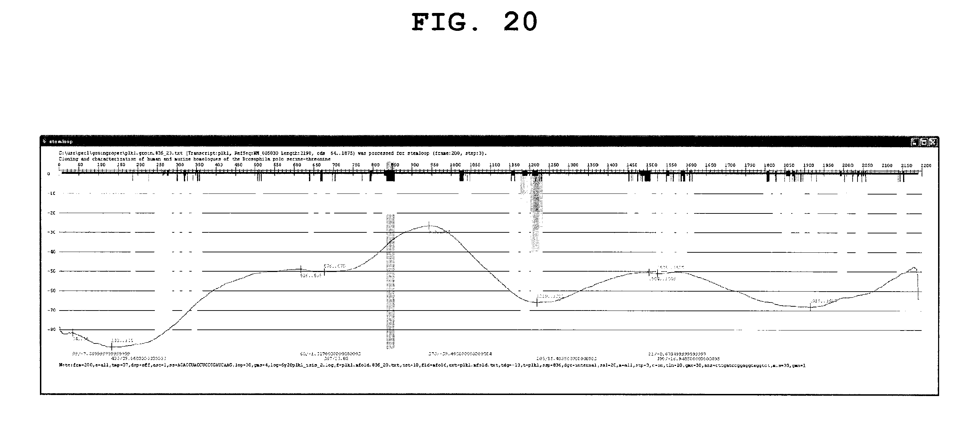

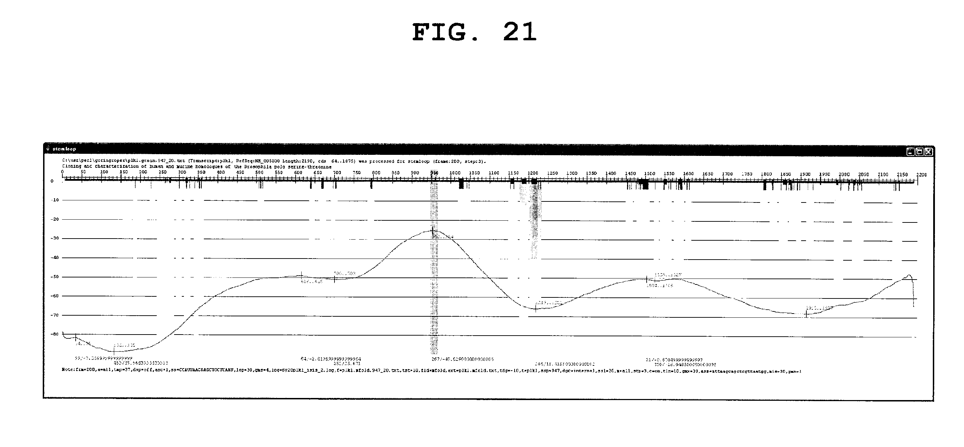

As a representative example of these, bar graphs visibly showing R-836(20) and R-947(20) are shown in Fig. 20 and Fig. 21, respectively.

In calculation of Dg value for obtaining a structure spectrum, structure prediction was conducted for each small-section covering one frame from one end of the objective section, and the lowest value of Gibbs free energy obtained for each small-section was regarded as a representative $\Delta$Grep for the small-section. For a certain base address x on the transcript, $\Delta$Grep of each small-section in which the base is involved was simply averaged and plotted as $\Delta$Gfr(x) in each graph in a color that is different from that of the graph bar (in the drawings of the present application, it is shown in black and white, however, actually it is clearly displayed for example, by a red curve in contrast to a blue graph bar). Concept of Gibbs free energy is a distribution of free energy found in the report "Secondary structure analysis in mouse cDNA" by Sadahiro Kumagai, in the annual report of Mori Foundation, 2001, Keio University. This number, which differs depending on selection of a parameter, is used for discussing whether the site forms a structure, as described in the report. However, the above report completely lacks description about discussion about what structure is concretely present and a method for conducting prediction.

The $\Delta$Gfr obtained in the manner as described above is a function of the position on the transcript calculated in a certain structure prediction condition. By integration for the entire length of the transcript, a value representing energy of the transcript calculated in the certain structure prediction condition:

$$\int \Delta Gfr(x)dx$$

can be obtained. In the above formula, "∫" is a integration notation.

Here, when the values obtained in different structure prediction conditions for the transcript, condition x and condition y are made as ∫ ΔG[x]fr(x)dx and ∫ ΔG[y]fr(y)dx, respectively, it is possible to determine energy difference between these two conditions x and y: ΔΔG[x-y]fr, by applying these to the following formula:

$$\Delta\Delta G[x-y]fr = \int \Delta G[x]fr(x)dx - \int \Delta G[y]fr(y)dx.$$

In other words, by conducting calculation for a certain structure prediction result X and a structure prediction result Y intended to be compared, it is possible to calculate energy change ΔΔGfr between these two structures. Concretely, by calculating ΔΔGfr from Rn which is a structure prediction result made on assumption that a certain antisense chain binds to a certain transcript, and R which is a structure prediction result in a state that no antisense chain binds, it is possible to calculate energy change for a case where a suitable structure for the antisense chain to act on the transcript is formed. In other words, making a structure naturally formed by the transcript be state (I), and a state that a binding site on the transcript is kept empty as a single strand for just binding to a certain antisense chain be state (II), energy lost in changing from the state (I) to the state (II): (ΔΔG[II-I]fr) is calculated. This calculation is executed for each antisense and the results are compared.

Provided that any of a plurality of antisense chains bind to the transcript with almost the same energy, it can be said that this ΔΔGfr indicates the tendency of binding of the antisense chain to the transcript to some extent (the number takes a positive value, and the smaller the number, at the higher tendency the antisense chain binds to the transcript).

However, in this case, since no correlation was observed between the activity of the antisense chain and the ΔΔGfr, it was suggested that "activity of the antisense chain used in the present invention is not controlled by tendency of binding to the transcript".

From the viewpoint of whether, when the antisense chain binds, RNase H can approach there, spectra of calculation results will be reviewed. For the sites in which addresses of the leading base are 1144, 1814 and 1865, the sites correspond to loop sites (1814, 1865) of a stem-loop structure or an interplace of a recess-shaped structure (1144), and are proved to be advantageous for approach of an enzyme.

On the other hand, the regions to which these bind are regions having ΔGfr of -50 or less.

Since a result satisfying these two points is not obtained from the "structure prediction result when an antisense chain exhibiting a inhibitory activity of 20% or less binds", it can be analyzed that for activity of the antisense chain used in the present invention,

(A) binding position of the antisense chain is in a loop site of the stem-loop structure having high probability after binding of the antisense chain, or in an interplace of the recess-shaped structure having high probability, and
(B) a region to which the antisense chain binds is a region where ΔGfr is -50 or less, are important.

Activity of the antisense chain is said to be RNA breakage occurring after recognition of a complex of an antisense chain and RNA by RNase H. Since the structure after binding of the antisense chain is important from the above analysis, activity of the antisense chain used in the present invention seems to depend on accessibility of RNase H. It is also suggested that activity of the antisense chain is largely influenced by degree of general structuring on a transcript. Primary action of the antisense chain strongly seems to be suppression of translation caused by a structure. Also for a transcript which is a target of antisense pharmaceuticals, a structure spectrum is obtained for various positions in the manner as described above on assumption that an antisense pharmaceutical binds thereto, and the effect thereof may be estimated in advance.

Reviewing the above structure prediction result R from a perspective of whether there is any other position where such an activity is much exerted, the region from base address 2063 to 2087 is a loop having a length allowing binding of an antisense chain. This is inherently a stem-loop having high probability and is a region having ΔGfr of -50 or less, and therefore it appears that the above requirements of (A) and (B) are well satisfied concurrently.

An antisense chain having "AGGGGGACAAGGCUGU" (SEQ ID NO: 1) or "GGGGGACAAGGCUGUA" (SEQ ID NO: 2) (base sequences respectively complementary to base sequences shown by base address 2065 to 2080 and 2064 to 2079 in a base sequence shown in SEQ ID NO: 4 of the sequence list in Patent document 2) targeting this region possibly exert an excellent inhibitory effect despite high GC content.

In this manner, the structure prediction method of the present invention provides a novel analysis means concerning mechanism of inhibitory activity of the antisense chain.

**Industrial utility**

As is descried in the description of background art, a local higher-order structure of RNA can be predicted by structure prediction software such as mfold. For a higher-order structure of particularly long RNA, since a spectroscopic analysis means such as X-ray crystal structure analysis cannot be substantially applied, there is no other way but to use structure prediction software for analyzing such a structure.

However, the structure prediction software has the drawbacks such as (i) reliable result is not provided for long sequence, (ii) presumption of prediction is far from the state in a cell. Furthermore, it is often the case that a result by the prediction software gives a plurality of candidates, which leads confusion.

In order to discuss the higher-order structure of long-chain RNA such as mRNA while overcoming the difficulty, there is a need of an analysis method that predicts a structure in a state that the prediction software provides as accurate result as possible, and leads a unified "spectrum" for the result.

As specifically described in the above, in the present invention, firs, long chain RNA is sectioned into frames, and the presence of a microstructure is predicted for a partial sequence in the frame, so that a prediction result of mfold or the like has certain reliability (only for the frame), and then to what extent a motif is actually present in the predicted specific position in the frame (that is, probability) is quantified as first probability Dg from the two points "stability (Gibbs free energy)" and "frequency (rate of existing in candidates extracted by the prediction software)", and further probability is determined similarly while shifting the frame by a constant length on RNA, and a specific position to which the first probability Dg is given is plotted on RNA sequence, and likelihood of actual presence of the motif in the specific position (secondary probability) for the entire long chain RNA is shown quantitatively by the overlapping degree of the same to give a unified "spectrum".

In the method of the present invention, it is predicted that RNA forms a motif in the specific position where both of these two numeral first and second probabilities are high. As a result, it is possible to predict a position of an intended secondary structure (motif) which is actually present on RNA at high probability, and to discuss it quantitatively.

Further, not only the stem-loop structure that is mainly focused conventionally, the presence of various secondary structures such as a valley structure (recess-shaped structure) sandwiched between two stem-loops may also be predicted quantitatively based on the first probability and the second probability.

The motif found in this manner has a possibility of being associated with biological phenomenon such that it is a target of miRNA, and may be used for drug discovery of antisense pharmaceuticals and low molecular weight pharmaceuticals by combination of the concept of ∆GTI.

This application is based on patent application No. 2006-337629 filed in Japan, and the contents thereof are hereby entirely incorporated by reference. In addition, the contents disclosed in any publication cited herein, including patents and patent applications, are hereby incorporated in their entireties by reference, to the extent that they have been disclosed herein.

**Claims**

1.  A method of predicting whether a specific secondary structure having desired characteristic parameter values is present in RNA, comprising the steps of:

    (A) selecting an objective section having entire length of L1 to be predicted from the entire length of RNA, and assuming a frame having entire length of L2 (L2<L1) which is longer than a sequence length of the specific secondary structure,
    in a small-section S1 which is defined to cover one frame from one end of the objective section, extracting a small structure which includes the specific secondary structure and can be formed by RNA, based on Gibbs free energy as an index for extraction and examining at which position in the extracted small structure, the specific secondary structure is present, thereby determining at what probability the specific secondary structure is present in each of at least one specific position X1 in the small-section S1 and making it as first probability Dg(X1) for each specific position X1 in the small-section S1,
    repeating the operation of defining a next small-section by shifting the frame toward the other end by constant length t (t<L2) and determining first probability for each specific position in the small-section similarly to that of the small-section S1 until the frame reaches the other end of the objective section, thereby obtaining first probabilities Dg(X1) to Dg(Xn) for each specific position in each small-section from the first small-section S1 to the last small-section Sn;

(B) applying each specific position determined for each small-section in the step of (A) to a corresponding position on the objective section, thereby determining at which position and at what degree on the objective section, each specific position overlaps, and making the degree of overlapping for each position on the objective section as second probability; and

(C) predicting presence of the specific secondary structure in the objective section based on the first probability and the second probability determined in the steps (A) and (B).

2. The method of claim 1, wherein the entire length L1 of the objective section is 100 or more bases.

3. The method of claim 1 or 2, wherein the specific secondary structure is a stem-loop structure, and the characteristic parameter values defining the structure include stem length, loop length, and an allowable number of mismatches in the stem-loop.

4. The method of any one of claims 1 to 3, wherein the specific secondary structure is a recess-shaped structure having an interspace existing between two stem-loop structures and not interacting with other base sequences, and respective inner stem parts of the stem-loop structures on both sides of the interspace, and the characteristic parameter values defining the structure include a number of bases in the interplace, and a sum of numbers of bases of the respective stem inner parts of the stem-loop structures on both sides.

5. The method of any one of claims 1 to 4, wherein a number of bases of the entire length L2 of the frame is 50 to 300.

6. The method of any one of claims 1 to 5, wherein a number of bases of the constant length t is 1 to 10.

7. The method of any one of claims 1 to 6, wherein in each small-section, extracting a small structure having Gibbs free energy of a predetermined value or less.

8. The method of any one of claims 1 to 7, wherein in the step (C), the first probability and the second probability are concurrently displayed in a graph in which the objective section corresponds to one axis, and the presence of the specific secondary structure in the objective section is predicted based on the first probability and the second probability displayed in the graph.

9. The method of claim 8, wherein the graph is a bar graph represented on an orthogonal coordinate plane in which the objective section corresponds to one axis and the value of the first probability corresponds to the other axis, and magnitude of the value of the first probability is displayed in correspondence with the length of the bar in the graph, and magnitude of the value of the second probability is displayed by changing color and/or pattern of the part where bars of the graph overlap with each other.

10. The method of claim 8 or 9, wherein for a sequence of the extracted specific secondary structure, calculating a value of Gibbs free energy of the specific secondary structure itself, and displaying the value in a superimposed manner on the graph.

11. The method of any one of claims 8 to 10, wherein for the extracted specific secondary structure,
a base usually not forming a base pair, and
a base not forming a base pair in one specific secondary structure but participating in formation of a base pair in other specific secondary structure are displayed in a superimposed manner in the graph so that they are discriminable from each other.

12. A computer program for predicting whether a specific secondary structure having intended characteristic parameter values is present in RNA, the program making a computer function as

(P1) an input means that inputs at least primary structure data of an objective section which is to be an object for prediction at least in primary structure of RNA, characteristic parameter values of the specific secondary structure, length L2 of a frame assumed as a section which is shorter than entire length L1 of the objective section and longer than a sequence length of the specific secondary structure, and constant length t(t<L2) which is a shifting amount of the frame;

(P2) a first calculation means that extracts a plurality of small structures which include the specific secondary structure and can be formed by RNA, based on Gibbs free energy as an index for extraction in a small-section S1 which is set to cover one frame from one end of the objective section, and examines at which position in the extracted small structure, the specific secondary structure is present, thereby determining at what probability the specific secondary structure is present in each of at least one specific position X1 in the small-section S1 and making it as first probability Dg(X1) for each specific position X1 in the small-section S1, and

repeats the operation of defining a next small-section by shifting the frame toward the other end by the constant length t (t<L2) and determining first probability for each specific position in the small-section similarly to that of the small-section S1, until the frame reaches the other end of the objective section, thereby obtaining first probabilities Dg(X1) to Dg(Xn) for each specific position in each small-section from the first small-section S1 to the last small-section Sn;

(P3) a second calculation means that applies each specific position determined for each small-section by the first calculation means to a corresponding position on the objective section, thereby determining at which position and at what degree on the objective section, each specific position overlaps, and making the degree of overlapping for each position on the objective section as second probability; and

(P4) an output means that outputs the first probability determined by the first calculation means and the second probability determined by the second calculation means.

13. The program of claim 12, wherein the entire length L1 of the objective section is 100 or more bases.

14. The program of claim 12 or 13, wherein the specific secondary structure is a stem-loop structure, and the characteristic parameter values defining the structure include stem length, loop length, and an allowable number of mismatches in the stem-loop.

15. The program of any one of claims 12 to 14, wherein the specific secondary structure is a recess-shaped structure having an interplace existing between two stem-loop structures and not interacting with other base sequences, and respective inner stem parts of the stem-loop structures on both sides of the interplace, and the characteristic parameter values defining the structure include a number of bases in the interplace, and a sum of numbers of bases of the respective stem inner parts of the stem-loop structures on both sides.

16. The program of any one of claims 12 to 15, wherein in each small-section, extracting a small structure having Gibbs free energy of a predetermined value or less.

17. The program of any one of claims 12 to 16, wherein the output means outputs the first probability and the second probability so that they are concurrently displayed in a graph in which the objective section corresponds to one axis.

18. The program of claim 17, wherein the graph is a bar graph represented on an orthogonal coordinate plane in which the objective section corresponds to one axis and the value of the first probability corresponds to the other axis, and magnitude of the value of the first probability is displayed in correspondence with the length of the bar in the graph, and magnitude of the value of the second probability is displayed by changing color and/or pattern of the part where bars of the graph overlap with each other.

19. The program of claim 17 or 18, wherein for a sequence of the extracted specific secondary structure, a value of Gibbs free energy of the specific secondary structure itself is calculated, and the value is displayed in a superimposed manner on the graph.

20. The program of any one of claims 17 to 19, wherein for the extracted specific secondary structure,
a base usually failing to form a base pair, and
a base failing to form a base pair in one specific secondary structure but participating in formation of a base pair in other specific secondary structure are displayed in a superimposed manner in the graph so that they are discriminable from each other.

21. An apparatus for predicting whether a specific secondary structure having intended characteristic parameter values is present in RNA, comprising

(M1) an input unit that inputs at least primary structure data of objective section which is to be an object for prediction at least in primary structure of RNA, characteristic parameter values of the specific secondary structure, length L2 of a frame assumed as a section which is shorter than entire length L1 of the objective section and longer than a sequence length of the specific secondary structure, and constant length t(t<L2) which is a shifting amount of the frame;

(M2) a first calculation unit that extracts a plurality of small structures which include the specific secondary structure and can be formed by RNA, based on Gibbs free energy as an index for extraction in a small-section S1 which is set to cover one frame from one end of the objective section, and examines at which position in the extracted small structure, the specific secondary structure is present, thereby determining at what probability the specific secondary structure is present in each of at least one specific position X1 in the small-section S1 and making it as first probability Dg(X1) for each specific position X1 in the small-section S1, and

repeats the operation of defining a next small-section by shifting the frame toward the other end by the constant length t (t<L2) and determining first probability for each specific position in the small-section similarly to that of the small-section S1 until the frame reaches the other end of the objective section, thereby obtaining first probabilities Dg(X1) to Dg(Xn) for each specific position in each small-section from the first small-section S1 to the last small-section Sn;

(M3) a second calculation unit that applies each specific position determined for each small-section by the first calculation unit to a corresponding position on the objective section, thereby determining at which position and at what degree on the objective section, each specific position overlaps, and making the degree of overlapping for each position on the objective section as second probability; and

(M4) an output unit that outputs the first probability determined by the first calculation unit and the second probability determined by the second calculation unit.

22. The apparatus of claim 21, wherein the entire length L1 of the objective section is 100 or more bases.

23. The apparatus of claim 21 or 22, wherein the specific secondary structure is a stem-loop structure, and the characteristic parameter values defining the structure include stem length, loop length, and an allowable number of mismatches in the stem-loop.

24. The apparatus of any one of claims 21 to 23, wherein the specific secondary structure is a recess-shaped structure having an interplace existing between two stem-loop structures and not interacting with other base sequences, and respective inner stem parts of the stem-loop structures on both sides of the interplace, and the characteristic parameter values defining the structure include a number of bases in the interplace, and a sum of numbers of bases of the respective stem inner parts of the stem-loop structures on both sides.

25. The apparatus of any one of claims 21 to 23, wherein at least the first calculation unit and the second calculation unit are a computer.

26. The apparatus of claim 25, wherein the input unit is included in the computer, and the primary structure data of the objective section is inputted by transferring the primary structure data stored in a storage device in or outside the computer in response to an instruction made by an operator who operates the computer, and the characteristic parameter values of a specific secondary structure, the frame length L2, and the constant length t are inputted by the operator who operates the computer.

# FIG. 1

# FIG. 2

(a)

q 1

q 2

(b)

q 1

q 2

(c)

q 1

(d)

q 1

# FIG. 3

(a)

(b)

# FIG. 4

Input

P 1

Database

P 2

P 3

P 4

Output

# FIG. 5

(E1)  (E2)  (E3)        (E4)        (E5)                (E6)  (E7)

FIG. 6

EP 2 096 567 A1

stemloop

C:\usr\perl\Groingroper\Survivin_HIT\_HIT000291947.3.stemloop2.txt (Transcript:_HIT000291947.3, RefSeq:NO Lengt
baculoviral IAP repeat-containing protein 5 isoform 1 [Homo sapiens] (BIRC5, Survivin).

FIG. 7

FIG. 8

# FIG. 9

FIG. 10

FIG. 11

FIG. 12

## FIG. 13

# FIG. 14

# FIG. 15

EP 2 096 567 A1

FIG. 16

38

FIG. 17

FIG. 18

# FIG. 19

EP 2 096 567 A1

# FIG. 20

FIG. 21

<table>
<tr><td colspan="2" align="center">**INTERNATIONAL SEARCH REPORT**</td><td>International application No.<br>PCT/JP2007/074063</td></tr>
</table>

**A. CLASSIFICATION OF SUBJECT MATTER**
*G06F19/00*(2006.01)i, *G06Q10/00*(2006.01)i, *C12N15/09*(2006.01)n

According to International Patent Classification (IPC) or to both national classification and IPC

**B. FIELDS SEARCHED**

Minimum documentation searched (classification system followed by classification symbols)
G06F19/00, G06Q10/00, C12N15/09

Documentation searched other than minimum documentation to the extent that such documents are included in the fields searched
Jitsuyo Shinan Koho            1922-1996   Jitsuyo Shinan Toroku Koho    1996-2008
Kokai Jitsuyo Shinan Koho     1971-2008   Toroku Jitsuyo Shinan Koho    1994-2008

Electronic data base consulted during the international search (name of data base and, where practicable, search terms used)
JMEDPlus(JDream2), JSTPlus(JDream2)

**C. DOCUMENTS CONSIDERED TO BE RELEVANT**

| Category* | Citation of document, with indication, where appropriate, of the relevant passages | Relevant to claim No. |
|---|---|---|
| A | Kiyoshi UCHIDA, "Antisense Kakusan no Bunshi Sekkei", Toa Gosei Kenkyu Nenpo TREND, 1998 first issue, 1998, pages 52 to 56 | 1-26 |
| A | Sadahiro KUMAGAI, "Mouse cDNA ni Okeru Niji Kozo Kaiseki", Keio Research Institute at SFC Mori Kikin Hokokusho (2001 Nendo), Internet <URL:http://www.kri.sfc.keio.ac.jp/report/mori/2001/e-syusi/e-21/> | 1-26 |
| A | Tateo TANAKA, "Kumiawase Saitekika Shuho ni yoru Pseudoknot Kozo o Fukunda RNA no Niji Kozo Yosoku", Information Processing Society of Japan Kenkyu Hokoku, 26 November, 1996 (26.11.96), Vol.96, No.118, pages 25 to 32 | 1-26 |

☒ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>06 March, 2008 (06.03.08) | Date of mailing of the international search report<br>18 March, 2008 (18.03.08) |
|---|---|
| Name and mailing address of the ISA/<br>    Japanese Patent Office | Authorized officer |
| Facsimile No. | Telephone No. |

Form PCT/ISA/210 (second sheet) (April 2007)

**INTERNATIONAL SEARCH REPORT**

| International application No. |
|---|
| PCT/JP2007/074063 |

C (Continuation).    DOCUMENTS CONSIDERED TO BE RELEVANT

| Category* | Citation of document, with indication, where appropriate, of the relevant passages | Relevant to claim No. |
|---|---|---|
| A | Michael Zuker, Mfold web server for nucleic acid folding and hybridization prediction, Nucleic Acids Research, 2003, Vol.31, No.13, p.3406-3415. | 1-26 |
| A | WO 2006/054788 A1  (Takeda Chemical Industries, Ltd.), 26 May, 2006 (26.05.06), Full text; all drawings (Family: none) | 1-26 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006054788 A
- WO 2004011610 A
- WO 2004011610 A2

**Non-patent literature cited in the description**

- *Proc. Natl. Acad. Sci. USA,* 1989, vol. 86, 7706-10
- **Sadahiro Kumagai.** Secondary structure analysis in mouse cDNA. *annual report of Mori Foundation,* 2001